# EUROPEAN PATENT SPECIFICATION

(11) **EP 0 665 859 B1**
(45) Date of publication and mention of the grant of the patent: **09.01.2002**
(21) Application number: 93924316.8
(22) Date of filing: 20.10.1993
(51) Int. Cl.: C08G 65/10, C08G 65/26, C08G 65/32

(54) **POLYMERIZATION OF CYCLIC ETHERS USING SELECTED METAL COMPOUND CATALYSTS**
POLYMERISATION VON CYCLISCHEN ETHERN UNTER ANWENDUNG DER KATALYSATOREN AUS GEWÄHLTEN METALLVERBINDUNGEN
POLYMERISATION D'ETHERS CYCLIQUES A L'AIDE DE LA CATALYSE PAR DES COMPOSES METALLIQUES CHOISIS

(30) Priority: 21.10.1992 US 964313; 23.02.1993 US 21368; 16.07.1993 US 93243
(43) Date of publication of application: 09.08.1995
(60) Divisional application: 00201220.1
(73) Proprietor: E.I. DU PONT DE NEMOURS AND COMPANY, Wilmington Delaware 19898 (US)
(72) Inventor: DRYSDALE, Neville, Everton, Newark, DE 19702-1026 (US); BOCKRATH, Richard, Edmund, Wilmington, DE 19806-1331 (US)
(74) Representative: Jones, Alan John
(86) International application number: US9309808
(87) International publication number: WO9409055

(56) References cited:
- WO-A-88/02661
- FR-A- 235 950
- US-A- 4 115 408
- US-A- 4 153 786
- POLYMER LETTERS ED., vol.14, 1976 pages 139 - 142 P. DREYFUSS ET AL.: 'Graft copolymers by oxonium polymerization'
- CHEMICAL ABSTRACTS, vol. 78, no. 16, 23 April 1973, Columbus, Ohio, US; abstract no. 98454j, MATSUKURA K. ET AL.: 'Polyether esters produced by using yttrium catalysts' & JP,A,7 231 438 (UNITIKA CO. LTD) 14 August 1972
- DATABASE WPI Derwent Publications Ltd., London, GB; AN 87-337120 & JP,A,62 240 319 (MITSUBISHI CHEM. IND. K.K.) 21 October 1987
- DATABASE WPI Derwent Publications Ltd., London, GB; AN 86-187083 & JP,A,61 120 832 (ASAHI CHEMICAL IND. K.K.) 7 June 1986

## Description

### FIELD OF THE INVENTION

This invention concerns the polymerization of oxiranes, oxetanes, oxepanes, 1,3-dioxolanes, 1,3,5-trioranes, and tetrahydrofurans to linear polyethers, catalyzed by selected metal compounds.

### BACKGROUND OF THE INVENTION

Cyclic ethers are polymerized by various means to give products of widespread utility. For instance, ethylene oxide is polymerized to polyethylene oxide which is useful, in lower molecular weight grades, for ceramics (as a binder), cosmetics, lubricants, polyurethanes; and in higher molecular weight grades, for packaging film, denture adhesives, lubricants, flocculation and for other articles and products. Tetrahydrofuran (THF) is polymerized to poly(tetramethylene ether) glycol which is useful in the preparation of Spandex fibers; polyurethane resins which are useful in elastomeric parts; and thermoplastic elastomers which are useful for molding various mechanical parts. Therefore, improved methods of making these polymers are sought. Also useful are methods of depolymerizing the polyethers to useful products, such as the cyclic ethers from which they were originally made. Such depolymerizations allow for the recycle of off specification or used polyethers to useful products such as polyethers, thereby reducing waste.

U.S. Patent 3,842,019 describes the polymerization of oxiranes and other small ring compounds by a presumed cationic mechanism, using as the catalyst the decomposition products of metal perfluoroalkylsulfonates. These catalysts are described as "latent", that is no reaction occurs until the metal salt is decomposed. The reactions reported are relatively slow, even at elevated temperatures.

WO-A-8802661 discloses a process for the polymerization of THF in the presence of aluminium tris-triflate wherein the reaction flask is protected from moisture and air and that gallium and boron perfluoroalkylenesulphonate are alternatives to the aforementioned aluminium salt.

U.S. Patents 5,084,586 and 5,124,417 describe the cationic polymerization of various monomers, including cyclic ethers, using onium cations, whose corresponding anions are fluororalkylsulfatometallates. Onium ion catalyzed cationic polymerizations are well known, and there is no mention in these patents of the use of metal salts not containing onium ions, such as metal triflates, as catalysts for the polymerization of cyclic ethers.

Japanese Patent Application 51-82397 describes the polymerization of tetrahydrofuran using a combination of fluorosulfonic acid and a carboxylic acid as catalysts. No mention is made of metal salts, such a metal triflates as catalysts.

J. S. Hrkach, et al., Macromolecules, vol. 23, p. 4042-4046 (1990) describe the polymerization of tetrahydrofuran using trimethylsilyl trifluoromethanesulfonate as the initiator. No mention is made of any other triflates as catalysts for this polymerization.

German Patent Application 2,459,163 describes the polymerization of THF using a combination of ferric chloride and carboxylic anhydride as catalyst.

G. A. Olah, et al., J. Appl. Polym. Sci., Vol. 45, 1355-1360 (1992) describe the use of boron, aluminum and gallium tristriflate to catalyze the polymerization of THF.

S. L. Borkowsky, et al., Organometal., Vol. 10, p. 1268-1274 (1991) report that certain zirconium complexes can initiate the polymerization of tetrahydrofuran. No mention is made of zirconium perfluoroalkylsulfonates, or of copolymers.

T. Misaki, et al., Nippon Kagaku Kaishi, p. 168-174 (1973) report on the polymerization of THF using a combination of metal aceylacetonates and acetyl chloride.

None of the metal compounds herein disclosed as catalysts are mentioned in the above references.

### SUMMARY OF THE INVENTION

This invention concerns a process for the polymerization of cyclic ethers, comprising, contacting one or more oxiranes, oxetanes, tetrahydrofurans, oxepanes, 1,3-dioxolanes or 1,3,5-trioxanes with a compound of the formula MZₛ·Qₜ, and an accelerator selected from the group consisting of carboxylic acids whose pKa in water is less than 6, carboxylic anhydrides and acyl halides, wherein:
M is a metal selected from the group consisting of cobalt, vanadium, niobium, tungsten, strontium, barium, scandium, yttrium, thulium and the other rare earth metals, titanium, zirconium, hafnium, chromium, silicon, molybdenum, tantalum, rhenium, iron, ruthenium, osmium, rhodium, iridium, palladium, platinum, gold, zinc, cadmium, mercury, copper, mischmetall, indium, germanium, tin, lead, arsenic; antimony and bismuth;
at least one of Z is an anion of the formula ⁻OSO₂R⁵, wherein R⁵ is perfluoroalkyl containing 1 to 12 carbon atoms or part of a fluorinated polymer wherein the carbon atoms alpha and beta to the sulfonate group are together bonded to at least four fluorine atoms, or tetraphenylborate, and the remainder of Z is oxo or one or more monovalent anions;
s is 2 when M is copper, strontium, barium, cobalt, rhodium, iridium, palladium, platinum, chromium, zinc, cadmium or mercury;
s is 3 when M is scandium, yttrium, thulium or other rare earth metal, arsenic, antimony, bismuth, gold, iron, ruthenium, osmium, indium or mischmetall;
s is 4 when M is titanium, zirconium, hafnium, silicon, molybdenum, germanium, tin, or lead;
s is 5 when M is rhenium, vanadium, niobium or tantalum;
s is 6 when M is tungsten;
Q is a neutral ligand;
t is 0 or an integer of 1 to 6; and
provided that each oxo group present counts as two of s.

### DETAILS OF THE INVENTION

In the polymerization process described herein one or more cyclic ethers, oxiranes, oxetanes, 1,3-dioxolanes, 1,3,5-trioxanes, or tetrahydrofurans are polymerized to form a polyether. Oxirane (more commonly called epoxide) is herein given its usual structure, a saturated three membered ring containing two carbon atoms and one oxygen atom. Oxetane is also given its common meaning, a saturated four membered ring containing three carbon atoms and one oxygen atom. The term oxepane means a saturated seven membered ring containing six carbon atoms and one oxygen atoms. The containing 3 oxygen atoms in which the oxygen atoms and carbons atoms are alternated. All of these terms include compounds containing those ring systems which are substituted with hydrocarbyl or hydrocarbylene groups containing 1 to 20 carbon atoms. The hydrocarbylene groups may form carbocyclic rings, which include bicyclic, tricyclic, etc., systems. By a hydrocarbylene group herein is meant a divalent radical containing carbon and hydrogen which is part of a carbocyclic ring.

Preferred cyclic ethers have the formula 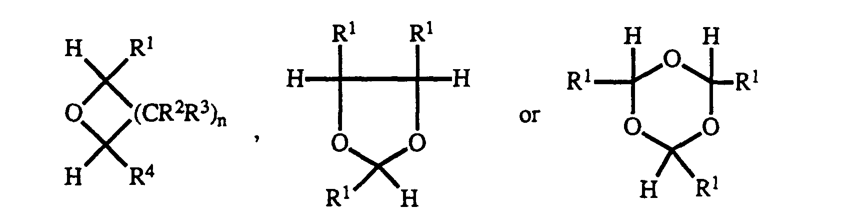 wherein n is 2 or 4 and each R¹, R², R³ and R⁴ is independently hydrogen or hydrocarbyl containing 1 to 20 carbon atoms. Some of these cyclic ethers polymerize to give repeat units of the formula -[CHR¹(CR²R³)ₙCHR⁴O]-. In a more preferred cyclic ether all of R¹, R², R³ and R⁴ are hydrogen. In another more preferred cyclic ether where n=2, R¹, one of R², both of R³ and R⁴ are hydrogen, and the remaining R² is alkyl containing 1-4 carbon atoms, especially preferably the remaining R² is methyl. By hydrocarbyl herein is meant a univalent radical containing carbon and hydrogen.

The polymerization is run in the presence of an accelerator. Suitable accelerators are carboxylic anhydrides, acyl halides, and carboxylic acids with a pkₐ of less than about 6 in water.

By a carboxylic anhydride is meant a compound containing the grouping -C(O)O(O)C-, wherein the free valencies are to other carbon atoms. A preferred carboxylic anhydride is an anhydride of an alkyl carboxylic acid or a halogen substituted alkyl carboxylic acid, and particularly preferred anhydrides are acetic anhydride and trifluoroacetic anhydride.

By an acyl halide is meant a compound containing the grouping -C(O)X, where X is chlorine or bromine and the free valence is to another carbon atom. In preferred acyl halides, X is chlorine. Preferred acyl halides are alkyl acyl halides, and especially preferred are acetyl halides, more preferably acetyl chloride.

By a carboxylic acid is meant a compound containing the grouping -C(O)OH, wherein the free valence is to another carbon atom. Preferred carboxylic acids have a pKa of less than 5 in water. Useful carboxylic acids include, but are not limited to acetic, trifluoroacetic, chloroacetic, benzoic, trichloroacetic, p-nitrobenzoic, butyric, formic, cyanoacetic, nitropropionic, acrylic, methacrylic, napthoic acids, N-acetylglycine and N-acetyltryptophan. Preferred carboxylic acids are trifluoroacetic, acetic, formic, cyanoacetic, nitropropionic, acrylic, methacrylic acids, N-acetylglycine and N-acetyltryptophan.

When carboxylic anhydride is present one half or more of the end groups are carboxylic esters. As is known to the artisan, these may be hydrolyzed to hydroxyl groups by reaction with water, preferably in the presence of a catalyst, such as a strong acid (sulfuric acid for instance) or a strong base (such as NaOH). The proportion of acetate ends increases the longer the polymerization is allowed to proceed. Although the polymeric diol is often the desired product (it can be used to make other polymers, such as polyurethanes and polyesters), the half ester or diester is also useful, as in relatively low molecular polymers which can be used as solvents.

When an acyl halide is used as the accelerator, the end groups are usually ester on one end, and the halide, X, on the other. Thus the complete formula for such a polymer could be X-[CHR¹(CR²R³)ₙCHR⁴O]-C(O)Y, where Y is the group to which the acyl group of the acyl halide was bound. Such polymers are useful as intermediates for the preparation of polymers containing different functional groups. For example, the ester may be hydrolyzed to a hydroxyl group, and the halide may be reacted to form another functional group such as nitrile. If a bis(acyl halide), X(O)CYC(O)X, is used as the accelerator, the product of the polymerization will be a polyether with halide (X) end groups which contains two internal ester groups, and may have the formula X- [CHR¹(CR²R³)ₙCHR⁴O]-C(O)YC(O)-[OCHR¹(CR²R³)ₙCHR⁴]-X . Useful bis (acyl halides) include adipoyl chloride, terephthaloyl chloride, and diglycolyl chloride [Cl(O)CCH₂OCH₂C(O)Cl].

When a carboxylic acid is used as the accelerator, both end groups are usually mostly ester. Thus the complete formula for such a polymer could be YO-[CHR¹(CR²R³)ₙCHR⁴O]-C(O)Y, where Y is the group to which the acyl group of the carboxylic acid was bound.

An important consideration in the preparation of polyethers is the number average molecular weight (Mn) of the polyether and its molecular weight distribution. When the polyether is to be used as a monomer in the preparation of another polymer (usually in the diol form), it is often preferred that the Mn of the polyether be in the range of about 400 to about 20,000, preferably about 500 to about 5,000.

The catalyst may be yttrium or rare earth compound of the formula MZ₃ where M is a trivalent ion of yttrium, or one of the rare earths, lanthanum, cerium, praeseodymium, neodymium, promethium, samarium, europium, gadolinium, terbium, dysprosium, holmium, erbium, thulium, ytterbium, and lutetium.

Preferred metals, M, are strontium, scandium yttrium, the rare earth metals, titanium, zirconium, hafnium, vanadium, niobium, tantalum, chromium, molybdenum, tungsten, rhenium, iron, ruthenium, palladium, copper, gold, zinc, tin and bismuth. More preferred metals are yttrium, the rare earth metals, and scandium. Especially preferred metals are yttrium, ytterbium, dysprosium, erbium, neodymium, lanthanum, and scandium. Another preferred metal is "mischmetall" (sometimes also called "didymium"), which is a mixture of rare earth metals as obtained from the ore.

It is believed monovalent anions that are relatively nonnucleophilic are useful as Z. Examples of such anions are tetraphenylborate, -OSO₂R⁵, wherein R⁵ is perfluoroalkyl, or wherein R⁵ is part of a fluorinated polymer wherein the carbon atoms alpha and beta to a sulfonate group are together bonded to at least 4 fluorine atoms (as in -CF₂CF₂OSO₂-). It is preferred if R⁵ is perfluoroalkyl. In a particularly preferred R⁵ is trifluoromethyl, and the anion is herein called "triflate".

Generally speaking, any metallic compound in which the correct metal in the correct oxidation state (see above) is present and bonded to a triflate or similar anion will be a catalyst. Such a compound must of course be reasonably stable during the polymerization (or depolymerization, see below), or decompose to another compound which is still a triflate (or similar anion) compound of the metal in the correct oxidation state. It has been found that, in general, the greater the number of triflate groups bonded to the metal cation, the more active the metal compound will be as a catalyst. It is preferred if half or more of the anions (Z) bound to each metal cation is triflate or a similar anion.

The metal catalysts may optionally contain one or more neutral ligands coordinated to the metal. By a neutral ligand is meant a neutral compound that can coordinate with the catalysts, usually the metal cation. Neutral ligands include water, and ethers such as dimethyl ether and tetrahydrofuran. Useful compounds containing neutral ligands include bis (n-cyclopentadienyl)tetrahydrofuran-bis (trifluoromethanesulfonato)-zirconium and bis (n-cyclopentadienyl) tetrahydrofuran-bis (trifluoromethanesulfonato) hafnium.

The metals catalysts may contain other anions than triflate and similar anions, and tetrafluoroborate, although at least one of triflate or tetrafluoroborate anions must be present. Some other useful anions are alkoxide, particularly lower alkoxide containing 1-4 carbon atoms, acetylacetonate, cyclopentadieneide, pentamethylcyclopentadieneide, t-butylacetylacetonate, and halide. It is preferred if all of the anions are triflate.

In general, the higher the molar ratio of metal compound to cyclic ether monomer originally present, the lower the molecular weight of the resulting polyether will be. Similarly, the higher the ratio of accelerator (if present) to monomer originally present, the lower the molecular weight of the polyether will be. It is believed the effects of these two ratios are cumulative. For these effects see Examples 7 and 8.

The polymerization may be run at a temperature of about -80°C to about 130°C. If this temperature is above the boiling point of the cyclic ether monomer, a pressure vessel may be used. A preferred temperature is ambient to the boiling point of the monomer, or 110°C, whichever is lower. An inert solvent such as di-n-butyl ether, diethyl ether or toluene may be used, but it is preferred if solvents are not present. Protic compounds such as water, methanol and ethanol should preferably not be present, and it is convenient to exclude them by drying the starting materials and keeping the process under an inert dry gas such as nitrogen. As in most chemical processes, the ingredients should be mixed at least initially. Continued agitation is preferred to assure that the process materials remain well mixed, and to avoid overheating. The polymerization is mildly exothermic. If the polymerization temperature goes up appreciably, refluxing of the monomer may be used to help cool the process.

The polymers produced often have polydispersities significantly less than 2, which is possibly indicative of a "Living polymerization". Also indicative of this is the fact that as the polymerization proceeds, the molecular weight, particularly the number average molecular weight, increases.

The polymerization processes can be done in a variety of ways known to the artisan. The polymerization can be done by batch, semi-batch and continuous processes. Continuous processes include continuous stirred tank reactor(s) with one or more stages, and/or plug flow reactors (see Example 19). Other variations will be evident to one skilled in this art.

In both the polymerization processes disclosed herein and the depolymerisation processes disclosed in WO-A-94/09055, the catalyst may be recovered and reused in either process. It may be recovered from the polymerization process by extracting the polymer formed with waters while it can be recovered from the depolymerization process by extracting the distillation residue with water. In both instances, the recovered catalyst may be used again in the polymerization process or in the depolymerization processes disclosed in WO-A-94/09055. In both instances the aqueous washings may be concentrated by removal of the water (as by evaporation) and the solid catalyst recovered. See Examples 20, and 28-32 for recovery and reuse of catalyst.

In the Examples, the following abbreviations are used:
GPC - gel permeation chromatography
Nafion™ - a sulfonated perfluoropolymer produced by E. I. du Pont de Nemours & Co., Wilmington, DE, USA
Mn - number average molecular weight
Mw - weight average molecular weight
RB - round bottom
PD - polydispersity (Mw/Mn)
PS - polystyrene
SS - stainless steel
STD - standard
Tf - triflate

### EXAMPLE 1

### Polymerization of THF with Yttrium Triflate and Acetic Anhydride

In a dry box, yttrium triflate (0.75 g) was added to each of three separate oven dried 100 mL RB flasks equipped with stirring bars. The flasks were sealed with rubber septa and removed from the dry box and nitrogen bleeds attached. THF (20 mL) followed by acetic anhydride (0.75 mL) were added to each flask. After 15, 30 and 45 minutes, a polymerization was terminated via the addition of 5% NaOH (10 mL) and THF (50 mL). The resulting organic phases were separated and concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 56.76 | 8180 | 17100 | 2.09 |
| 30 mins. | 67.02 | 6630 | 14600 | 2.20 |
| 45 mins. | 73.11 | 6210 | 13300 | 2.02 |

### EXAMPLE 2

### Polymerization of THF with Ytterbium Triflate and Acetic Anhydride

In a dry box, ytterbium triflate (0.75 g) was added to each of four separate oven dried 100 mL RB flasks equipped with stirring bars. The flasks were sealed with rubber septa and removed from the dry box.
Nitrogen bleeds were attached and THF (20 mL) followed by acetic anhydride (0.75 mL) were added to each flask. After 15, 30, 45 and 60 minutes, a polymerization was terminated via the addition of 5% NaOH (10 mL) and THF (50 mL). The resulting organic phases were separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 56.09 | 8400 | 16200 | 1.93 |
| 30 mins. | 67.98 | 7360 | 14900 | 2.03 |
| 45 mins. | 69.67 | 5890 | 13100 | 2.22 |
| 60 mins. | 71.31 | 6010 | 12800 | 2.15 |

### EXAMPLE 3

### Polymerization of THF with Dysprosium Triflate and Acetic Anhydride

In a dry box, dysprosium triflate (0.75 g) was added to each of four separate oven dried 100 mL RB flasks equipped with stirring bars. The flasks were sealed with rubber septa and removed from the dry box. After the attachment of nitrogen bleeds THF (20 mL) followed by acetic anhydride (0.75 mL) were added to each flask. After 15, 30, 45 and 60 minutes, a polymerization was terminated via the addition of 5% NaOH (10 mL) and THF (50 mL). The resulting organic phases were separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 52.03 | 7260 | 15700 | 2.17 |
| 30 mins. | 63.86 | 7220 | 15700 | 2.18 |
| 45 mins. | 70.05 | 6250 | 14300 | 2.30 |
| 60 mins. | 71.36 | 6010 | 13700 | 2.29 |

### EXAMPLE 4

### Polymerization of THF with Erbium Triflate and Acetic Anhydride

In a dry box, erbium triflate (0.75 g) was added to each of four separate oven dried 100 mL RB flasks equipped with stirring bars. After sealing with rubber septa the flasks were removed from the dry box.
Nitrogen bleeds were attached and THF (20 mL) followed by acetic anhydride (0.75 mL) were added to each flask. After 15, 30, 45 and 60 minutes, a polymerization was terminated via the addition of 5% NaOH (10 mL) and THF (50 mL). The resulting organic phases were separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 52.82 | 8460 | 15900 | 1.89 |
| 30 mins. | 62.96 | 7390 | 17100 | 2.32 |
| 45 mins. | 66.79 | 8070 | 16400 | 2.04 |
| 60 mins. | 68.20 | 7250 | 16100 | 2.22 |

### EXAMPLE 5

### Polymerization of THF with Lanthanum Triflate and Acetic Anhydride

In a dry box, lanthanum triflate (0.75 g) was added to each of four separate oven dried 100 mL RB flasks equipped with stirring bars. The flasks were sealed with rubber septa and then removed from the dry box. Nitrogen bleeds were attached and THF (20 mL) and acetic anhydride (0.75 mL) were added to each flask. After 15, 30, 45 and 60 minutes, a polymerization was terminated via the addition of 5% NaOH (10 mL) and THF (50 mL). The resulting organic phases were separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 5.60 | 9780 | 13900 | 1.42 |
| 30 mins. | 11.27 | 13700 | 20900 | 1.53 |
| 45 mins. | 40.30 | 17000 | 28100 | 1.65 |
| 60 mins. | 59.24 | 15800 | 33400 | 2.11 |

### EXAMPLE 6

### Polymerization of THF with Neodymium Triflate and Acetic Anhydride

In a dry box, to an oven dried 100 mL RB flask equipped with a stirring bar was added neodymium triflate (0.75 g). The flask was sealed with a rubber septum and removed from the dry box. A nitrogen bleed was attached and THF (20 mL) followed by acetic anhydride (0.75 mL) were added. After 30 minutes the polymerization was terminated via the addition of 5% NaOH (10 mL) and THF (50 mL). The resulting organic phase was separated, concentrated at reduced pressure and then dried under vacuum yielding 7.56 g (42.6%) of polymer. GPC analysis: Mn = 8460, Mw = 22300, PD = 2.65 (PS STD).

### EXAMPLE 7

### Polymerization of THF with Yttrium Triflate and Acetic Anhydride

In a dry box, yttrium triflate (0.75 g) was added to each of three oven dried 100 mL RB flasks equipped with stirring bars. The flasks were sealed with rubber septa and removed from the dry box. Nitrogen bleeds were attached and THF (20 mL) added to each flasks. Acetic anhydride (0.25, 0.50 and 0.75 mL) was added respectively to each flask. After 60 minutes the polymerizations were quenched via the addition of 5% NaOH (10 mL) and THF (50 mL), the resulting organic phases were separated, concentrated at reduced pressure and then dried in vacuo overnight. Polymer yields and GPC analysis:

| Acetic Anhydride | Polymer Yield (%) | Mn (PS STD) | Mw | PD |
|---|---|---|---|---|
| 0.25 mL | 75.02 | 8080 | 18100 | 2.25 |
| 0.50 mL | 73.33 | 6940 | 14900 | 2.15 |
| 0.75 mL | 75.20 | 5080 | 13600 | 2.68 |

### EXAMPLE 8

### Polymerization of THF with Yttrium Triflate and Acetic Anhydride

In a dry box, to three 100 mL RB flasks equipped with stirring bar were added 0.25, 0.50 and 1.00 g yttrium triflate respectively. The flask were sealed with rubber septa and removed from the dry box.
Nitrogen bleeds were attached and THF (20 mL) and acetic anhydride (1.00 mL) were added to each flask. After 60 minutes the polymerizations were terminated via the addition of 5% NaOH (10 mL) and THF (50 mL). The resulting organic phases were separated, concentrated at reduced pressure and dried under vacuum overnight. Polymer yields and GPC analysis:

| Yttrium Triflate | Polymer Yield (%) | Mn (PS STD) | Mw | PD |
|---|---|---|---|---|
| 0.25 g | 50.11 | 11300 | 26200 | 2.02 |
| 0.50 g | 70.79 | 8060 | 17600 | 2.16 |
| 1.00 g | 81.96 | 4820 | 10500 | 2.09 |

### EXAMPLE 9

### Polymerization of THF with Yttrium Triflate and Acetic Anhydride in Diethyl Ether

In a dry box, yttrium triflate (0.75 g) was weighed into an oven dried 100 mL RB flask equipped with stirring bar. A reflux condenser was attached and the apparatus sealed with a rubber septum and removed from the dry box. A nitrogen bleed was attached and diethyl ether (20 mL), THF (20 mL) and acetic anhydride (0.75 mL) were added. After 60 minutes the polymerization was quenched via the addition of 5% NaOH (10 mL) and diethyl ether (50 mL). The resulting organic phase was separated, concentrated and dried under vacuum. Yield: 3.86 g (21.76%). GPC analysis: Mn = 2960, Mw = 7800, PD = 2.63 (PS STD).

### EXAMPLE 10

### Polymerization of THF with Yttrium Triflate and Acetic Anhydride in Toluene

In a dry box, yttrium triflate (0.75 g) was weighed into an oven dried 100 mL RB flask equipped with a stirring bar. After sealing with a rubber septum, removal from the dry box, and attachment of a nitrogen bleed, toluene (20 mL), THF (20 mL) and acetic anhydride (0.75 mL) were added. After 60 minutes the polymerization was terminated via the addition of 5% NaOH (10 mL) and toluene (50 mL). The resulting organic phase was separated, concentrated at reduced pressure and then dried under vacuum. Yield: 1.73 g (9.75%). GPC analysis: Mn = 1150, Mw = 2700, PD = 2.34 (PS STD).

### EXAMPLE 11

### Copolymerization of THF/3-Methyl-THF with Yttrium Triflate and Acetic Anhydride

In a dry box, yttrium triflate (0.75 g) was added to each of three separate oven dried 100 mL RB flasks equipped with stirring bars. The flasks were sealed with rubber septa and removed from the dry box wherein nitrogen bleeds were attached. THF (15 mL) and 3-methyl-THF (5 mL) followed by acetic anhydride (0.75 mL) were added to each flask. After 15, 30 and 45 minutes, a polymerization was terminated via the addition of 5% NaOH (10 mL) and THF (50 mL). The resulting organic phases were separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 39.50 | 6920 | 12400 | 1.80 |
| 30 mins. | 51.63 | 6280 | 13200 | 2.11 |
| 45 mins. | 57.27 | 5860 | 12700 | 2.17 |

¹H NMR analysis showed ∼12-13% incorporation of 3-methyl-THF in the polymers.

### EXAMPLE 12

### Polymerization of THF with Yttrium Triflate and Trifluoroacetic Anhydride

In a dry box, yttrium triflate (0.75 g) was weighed in an oven dried 100 mL RB flask equipped with a stirring bar. After sealing with a rubber septum and removal from the dry box and attachment of a nitrogen bleed THF (20 mL) was added followed by trifluoroacetic anhydride (3.00 mL). After 2 hrs. the polymerization was quenched by the addition of 5% NaOH (10 mL) and THF (50 mL). Diethyl ether (50 mL) was added to effect separation of the organic/aqueous phase. The organic phase was separated, concentrated at reduced pressure and then dried under vacuum. Yield: 5.40 g (30.44%). GPC analysis: Mn = 53900, Mw = 86200, PD = 1.78 (PS STD).

### EXAMPLE 13

### Polymerization of THF with Ytterbium Triflate and Propionic Anhydride

In a dry box, ytterbium triflate (1.00 g) was weighed into an oven dried 100 mL RB flask equipped with a stirring bar. The flask was stoppered with a rubber septum and removed from the dry box and a nitrogen bleed was attached. THF (20 mL) and propionic anhydride (1.00 mL) were added via syringes. After 60 minutes the polymerization was quench with 5% NaOH (10 mL) and THF (50 mL). The resulting organic phase was separated, concentrated at reduced pressure and then dried in vacuo. Yield: 12.69 g (71.5%). GPC analysis: Mn = 6520, Mw = 14500, PD = 2.23.

### EXAMPLE 14

### Polymerization of 3-Methyl-THF with Yttrium Triflate and Acetic Anhydride

In a dry box, yttrium triflate (0.75 g) was weighed into an oven dried 100 mL RB flask equipped with a stirring bar. The flask was sealed with a rubber septum and removed from the dry box. A nitrogen bleed was attached and 3-methyl-THF (20 mL) was added followed by acetic anhydride (0.75 g). After stirring overnight the polymerization was terminated by the addition of 5% NaOH (10 mL) and THF (25 mL). The resulting organic phase was separated, concentrated at reduced pressure and dried under vacuum. Yield: 6.12 g (34.50%). GPC analysis: Mn = 437, Mw = 808, PD = 1.85.

### EXAMPLE 15

### Polymerization of THF with Yttrium Triflate and Acetic Anhdride

In a dry box, yttrium triflate (0.75 g) was weighed into an oven dried 100 mL RB flask equipped with a stirring bar. After sealing with a rubber septum the flask was removed from the dry box and a nitrogen bleed attached. THF (20 mL) and acetic anhydride (1.00 mL) were added. After 17.5 hrs. THF (20 mL) and acetic anhydride (1.00 mL) were added to the thick viscous solution. After an additional 2 hrs THF (20 mL) and acetic anhydride were again added to the polymerized solution. The polymerization was terminated 2.5 hrs later via the addition of 5% NaOH (20 mL) and THF (100 mL). The organic phase was separated, concentrated at reduced pressure and dried under vacuum. Polymer yield: 32.3 g (61.23%). GPC analysis: Mn = 2490, Mw = 8440, PD = 3.39 (PS STD).

### EXAMPLE 16

### Polymerization of THF with Ytterbium Triflate

In a dry box, ytterbium triflate (1.00 g) was weighed in a 100 mL RB flask equipped with a stirring bar. After sealing with a rubber septum the flask was removed from the dry box and a nitrogen bleed attached. THF (20 mL) was then added via syringe. The polymerization was allowed to proceed overnight and then terminated via the addition of H₂O (25 mL) and diethyl ether (75 mL). The organic phase was separated, concentrated at reduced pressure and then dried under vacuum. Polymer yield: 0.520 g (2.93%). GPC analysis: Mn = 416000, Mw = 842000, PD = 2.02 (PS STD).

### EXAMPLE 17

### Polymerization of 7-Oxabicyclo [2.2.1] heptane with Ytterbium Triflate and Acetic Anhydride

In a dry box, ytterbium triflate (0.5 g) was weighed into a 100 mL RB flask equipped with a stirring bar. After sealing with a rubber septum, the flask was remove from the dry box and a nitrogen bleed attached. 7-Oxabicyclo[2.2.1]heptane (10 mL, distilled from potassium carbonate) was added followed by acetic anhydride (0.5 mL). After 1 hr. the polymerization was terminated by the addition of 5% NaOH (10 mL), THF (75 mL) and diethyl ether (∼50 mL). The organic phase was separate, concentrated at reduced pressure and then dried under vacuum. Polymer yield: 1.00 g. GPC analysis: Mn = 233, Mw = 522, PD = 2.24 (PS STD).

### EXAMPLE 18

### Polymerization of Cyclohexene Oxide with Lanthanium Triflate

In a dry box, lanthanum triflate (0.75 g) was weighed in a oven dried 100 mL three neck flask equipped with a stirring bar, reflux condenser and addition funnel. Toluene (20 mL) was added via syringe and cyclohexene oxide (20 mL) was slowly added via the addition funnel. The polymerization was terminated after 2.75 hrs. via the addition of H₂O (10 mL) and toluene (100 mL). The organic phase was separated, concentrated at reduced pressure and then dried under vacuum. Polymer yield: 12.4 g (63.9%). GPC analysis (bimodal distribution): Mn = 4510, Mw = 25700, PD = 5.70 (PS STD).

### EXAMPLE 19

### Continuous Polymerization of THF with Ytterbium Triflate and Acetic Anhydride

A solution of THF (∼500 mL) and ytterbium triflate (25 g) was charged into a 500 mL capacity ISCO pump, which was connected to a 3 way 3.2 mm SS connector ("T" mixer) via 8 cm of 3.2 mm SS tubing containing a check valve. A second ISCO pump (500 mL capacity) was charged with ∼100 mL of acetic anhydride and this was connected to the "T" mixer by 75 cm of 3.2 mm SS tubing also containing a check valve. The feed rate of the THF/ytterbium triflate solution was 3.3 mL/min and that of the acetic anhydride was 0.25 mL/min. The "T" mixer was connected to a glass stirred holdup tank (approximately 60 mL volume) by 12 cm of 3.2 mm SS tubing. This tank was then connected to a second stirred holdup tank (approximately 55 mL volume) via Cajon flex tubing with ultra torr fitting (6.4 mm, 13 cm). This in turn was connected to a third glass reactor, plug flow (approximately 60 mL volume), again via Cajon flex tubing with ultra torr fitting (6.4 mm, 13 cm). The polymerized solution exiting from the third reactor was fed to a stirred beaker containing water/diethyl ether. Each reactor was equipped with thermal well port. During the polymerization the temperature in the first reactor stabilized to 41-42°C and that of the second reactor to 31-32°C and that of the third reactor 26-27°C. After the contents of the THF/ytterbium triflate pump was discharged, and two fractions of polymer were collected, the pump was again refilled with a solution of THF (∼500 mL) and ytterbium triflate (25 g). Three fractions were collected. The last fraction was obtained by purging the system with diethyl ether.

The organic phases were separated, concentrated at reduced pressure and then dried under vacuum giving the following:

| Fraction | Weight |
|---|---|
| 1 | 106.86 g |
| 2 | 79.59 g |
| 3 | 56.97 g |
| 4 | 220.2 g |
| 5 | 97.2 g |

The aqueous phases from above were collected, concentrated at reduced pressure and then dried under vacuum at 180°C giving a cream solid, 46.98 g, representing a 93.94% recovery of the total ytterbium triflate catalyst used.

### EXAMPLE 20

### Polymerization of THF with Ytterbium Triflate (Recovered from Example 19) and Acetic Anhydride

In a dry box, ytterbium triflate (1.00 g), recovered catalyst from Example 19, was weighed out in a 100 mL RB flask equipped with a stirring bar. A rubber septum was attached and the flask removed from the dry box. A nitrogen bleed was attached and THF (20 mL) added followed by acetic anhydride (1.00 mL). After 1 hr. the polymerization was terminated by the addition of water (25 mL), THF (25 mL) and diethyl ether (50 mL), the resulting organic phase was separated, concentrated at reduced pressure, then dried under vacuum affording 13.42 g (75.65%) of polymer.

### EXAMPLE 21

### Polymerization of THF with Yttrium Triflate and Acetic Andyride at -78°C

In a dry box, yttrium triflate (0.75 g) was weighed in an oven dried 100 mL RB flask equipped with a stirring bar. After sealing with a rubber septum, removal from the dry box and the attachment of a nitrogen bleed, THF (20 mL) was added. The resulting mixture was cooled to -78°C. Acetic anhydride (0.75 mL) was then added, the polymerization was terminated 5 hrs. later by the addition of water (25 mL) and diethyl ether (50 mL). After warming to room temperature the resulting organic phase was separated, concentrated at reduced pressure, then dried under vacuum affording 0.58 g (3.27%) of polymer.

### EXAMPLE 22

### Preparation of Didymium (Mischmetall) Triflate

Didymium (mischmetall) oxide (17 g) and water (50 mL) were added to a 200 mL RB flask equipped with stirring bar and an addition funnel and reflux condenser. Triflic acid (50 g) was slowly added via the addition funnel to the resulting stirred slurry. After the addition was completed a homogeneous solution resulted, thus an additional 2.0 g of the oxide was added and the slurry heated to reflux for 2 hrs. The cooled slurry was filtered, the filtrate concentrated at reduced pressure and then dried under vacuum at 150-210°C affording 58.4 g of a pink solid.

### EXAMPLE 23

### Polymerization of THF with Didymium (Mischmetall) Triflate: Polymerization Time on Polymer Yield

In a dry box, didymium triflate (0.75 g) was added to each of four separate oven dried 100 mL RB flasks equipped with stirring bars. The flasks were sealed with rubber septa and removed from the dry box and nitrogen bleeds attached. THF (20 mL) followed by acetic anhydride (0.75 mL) were added to each flask. After 15, 30, 45 and 60 minutes, a polymerization was terminated via the addition of 5% NaOH (10 mL) and THF (50 mL). The resulting organic phases were separated, concentrated at reduced pressure and then dried under vacuum. Polymer yields:

| Polymer Time | Polymer Yield (%) |
|---|---|
| 15 mins. | 13.92 |
| 30 mins. | 34.94 |
| 45 mins. | 43.74 |
| 60 mins | 49.4 |

### EXAMPLE 24

### Polymerization of Refluxing THF with Yttrium Triflate and Acetic Anhydride

In a dry box, yttrium triflate (0.75 g) was weighed into an oven dried 100 mL flask equipped with a stirred bar, a reflux condenser was attached, the flask sealed with rubber septum and removed from the dry box and a nitrogen bleed attached. THF (20 mL) was added and the resulting mixture heated to reflux via an oil bath (temp. ∼80°C). Acetic anhydride (0.75 mL) was added to the stirred refluxing mixture. After 30 minutes the polymerization was terminated via the addition of 5% NaOH (10 mL) and THF (50 mL). The cooled organic phase was separated, concentrated at reduced pressure, then dried under vacuum giving 6.44 g (36.30%) of polymer.

### EXAMPLE 25

### Preparation of Ytterbium Nafion® Salt

In a 300 mL RB flask were added ytterbium oxide (0.75 g) and Nafion® perfluorinated ion exchange resin powder (300 mL, 5 wt. % solution in a mixture of lower aliphatic alcohols and 10% water). The resulting mixture was heated to 100°C and stirred overnight. The resulting solution was filtered and dried under vacuum at 150°C, affording 9.21 g of a light brown solid.

### EXAMPLE 26

### Polymerization of THF with Ytterbium Nafion® Salt and Acetic Anhydride

In a dry box, the ytterbium Nafion® salt (1.00 g, from Example 25) was added to each of four oven dried 100 mL RB flasks equipped with stirring bars. The flasks were sealed with rubber septa and removed from the dry box and nitrogen bleeds attached. THF (20 mL) followed by acetic anhydride (1.00 mL) were added to each flask. After 2, 3, 4 and 5 hrs. a polymerization was terminated by the addition of water (25 mL) and diethyl ether (50 mL). The organic phases were separated, concentrated at reduced pressure and then dried under vacuum to give the following:

| Polymer Time | Polymer Yield (%) |
|---|---|
| 2 hrs. | 5.24 |
| 3 hrs. | 11.39 |
| 4 hrs. | 17.08 |
| 5 hrs. | 22.66 |

### EXAMPLE 27

### (A) Depolymerization of PolyTHF with Yttrium Triflate

Polytetrahydrofuran 1000 (300 g, Aldrich) and yttrium triflate (9 g) were placed in a 500 mL three neck flask equipped with a stirring bar, Vigreaux column (30.5 cm) and a fractional distillation head. A nitrogen purge was then attached and all other openings glass stoppered. The resulting mixture was then heated by an oil bath and the water clear distillate fractions collected as follows:

| Fraction | Oil Bath Temp (°C) | Head Temp (°C) | Weight |
|---|---|---|---|
| 1 | 171-175 | 64.5 | 67.49 |
| 2 | 176 | 64.5 | 71.84 |
| 3 | 176 | 64.5 | 32.84 |
| 4 | 178 | 64.5 | 58.67 |
| 5 | 178 | 64.5 | 56.71 |

Total weight of distillate collected: 287.55
¹ H NMR analyses of all five fractions confirmed the product to be THF.
Yield (Recovery): 95.85%

### (B) Polymerization of Recovered THF with Ytterbium Triflate and Acetic Anhydride

In a dry box, ytterbium triflate (1.00 g) was added to an oven dried 100 mL flask equipped with a stirring bar. The flask was then sealed with a rubber septum and removed from the dry box and a nitrogen purge attached. Tetrahydofuran (20 mL) from the fourth fraction of Example 27(A) was added followed by 1 mL of acetic anhydride. After 1 hour no polymerization was apparent, thus an additional 1 mL of acetic anhydride was added. After 1 hour the polymerization was terminated via the addition of 5% NaOH and THF (50 mL), the organic phase separated, concentrated at reduced pressure and then dried in vacuo overnight affording 10.31 g (58%) of polymer. GPC analysis: Mn = 1970, Mw = 6650, PD = 3.38 (PS STD).

### EXAMPLE 28

### Polymerization of Recovered Purified THF with Ytterbium Triflate and Acetic Anhydride

Fractions 2-4 of experiment Example 27(A) were combined and distilled from sodium/benzophenone. Twenty mL of this dried THF was added to ytterbium triflate (1 g), previously weighed out in an oven dried 100 mL flask equipped with stirring bar and a nitrogen purge. Acetic anhydride (1 mL) was then added, after 1 hour the polymerization was terminated via the addition of 5% NaOH and THF (50 mL), the organic phase separated and concentrated at reduced pressure and then in vacuo, affording 13.32 g (78.08%) of polymer. GPC analysis: Mn = 4110, Mw = 8860, PD = 2.15 (PS STD).

### EXAMPLE 29

### (A) Depolymerization of Poly-THF with Yttrium Triflate: Reuse of Catalyst

To the residue remaining from Example 27(A) was added polytetrahydrofuran 1000 (300 g, Aldrich). The apparatus was reassembled, the resulting mixture heated by an oil bath, and the resulting water clear distillate fractions were collected as follows:

| Fraction | Oil Bath Temp (°C) | Head Temp (°C) | Weight |
|---|---|---|---|
| 1 | 170-174 | 63-64 | 43.39 |
| 2 | 174 | 64 | 62.68 |
| 3 | 175 | 65 | 66.15 |
| 4 | 177 | 65 | 55.15 |
| 5 | 177 | 65 | 32.58 |

Total weight of distillate collected: 259.95 g
Yield (Recovery): 86.65%
Total time elapsed from start of collection to termination of Example: 2 hrs. 50mins.

### (B) Recovery of Catalyst from Depolymerization

Water (100 mL) was added to the residue from Example 29(A), the resulting mixture was stirred at room temperature for approximately 1 hour, the aqueous phase separated and concentrated at reduced pressure and dried in vacuo at 180°C overnight affording a brown solid. This solid was again dissolved in water, then filtrated, the filtrate concentrated at reduced pressure. The resulting solid was dried under vacuum at 180°C overnight affording a cream solid: 6.48 g (72%) of recovered catalyst.

### (C) Activity of recovered Catalyst in the Polymerization of THF

In a dry box, the recovered catalyst of Example 29(B) (1 g) was placed in an oven dried 100 mL flask equipped with a stirring bar. The flask was sealed with a rubber septum and removed from the dry box and a nitrogen purge attached. THF (20 mL) was then added followed by acetic anhydride (1 mL). After 1 hour the polymerization was terminated via the addition of 5% NaOH and THF (50 mL), the organic phase separated, concentrated and dried in vacuo overnight affording 13.86 g (78.13%) of polymer. GPC Analysis: Mn = 4460, Mw = 9280, PD = 2.08 (PS STD).

### EXAMPLE 30

### Polymerization of THF with Bis (n-cyclopentadienyl)-tetrahydrofuran-bis (trifluoromethanesulfonato)-zirconium and Acetic Anhydride

In a dry box, bis (*n*-cyclopentadienyl) tetrahydrofuran-bis (trifluoromethanesulfonato) zirconium (0.50 g) was added to each of four separate oven dried 100 mL RB flasks equipped with stirring bars. The flasks were sealed with rubber septa and removed from the dry box. After the attachment of nitrogen bleeds THF (10 mL) and acetic anhydride (0.50 mL) were added to each flask. After 15, 30, 45 and 60 minutes, a polymerization was terminated via the addition of water (25 mL), THF (50 mL) and ether (25 mL). The resulting organic phases were separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 39.34 | 12700 | 14100 | 1.11 |
| 30 mins. | 54.79 | 15000 | 19000 | 1.27 |
| 45 mins. | 63.92 | 16000 | 22100 | 1.38 |
| 60 mins. | 64.26 | 17200 | 24500 | 1.41 |

### EXAMPLE 31

### Polymerization of THF with Bis (n-cyclopentadienyl)bis (trifluoromethanesulfonato) titanium and Acetic Anhydride

In a dry box, bis (n-cyclopentadienyl)-bis (trifluoromethanesulfonato) titanium (0.50 g) was added to each of four separate oven dried 100 mL RB flasks equipped with stirring bars. The flasks were sealed with rubber septa and then removed from the dry box. Nitrogen bleeds were attached and THF (10 mL) and acetic anhydride (0.50 mL) were added to each flask. After 15, 30, 45, 70 minutes a polymerization was terminated via the addition of water (25 mL), ether (25 mL) and THF (50 mL). The separated organic phases were washed repeatedly with water (3 x 25 mL), then separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 39.35 | 10700 | 12000 | 1.12 |
| 30 mins. | 61.33 | 13900 | 17300 | 1.25 |
| 45 mins. | 67.08 | 14200 | 19300 | 1.35 |
| 70 mins. | 65.50 | 12400 | 19300 | 1.56 |

### EXAMPLE 32

### Polymerization of THF with Bis (n-cyclopentadienyl) tetrahydrofuran-bis (trifluoromethanesulfonato) zirconium and Acetyl Chloride

In a dry box, bis (*n*-cyclopentadienyl) tetrahydrofuran-bis (trifluoromethanesulfonato) zirconium (0.50 g) was added to each of three separate oven dried 100 mL RB flasks equipped with stirring bars. The flasks were sealed with rubber septa and removed from the dry box. After the attachment of nitrogen bleeds, THF (10 mL) and acetyl chloride (0.375 mL) were added to each flask. After 15, 30, and 45 minutes, a polymerization was terminated via the addition of water (25 mL), THF (50 mL) and ether (25 mL). The resulting organic phases were washed repeatedly with water (3 x 25 mL), separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 59.86 | 7980 | 10800 | 1.35 |
| 30 mins. | 68.88 | 7470 | 11000 | 1.48 |
| 45 mins. | 68.65 | 5620 | 9920 | 1.76 |

### EXAMPLE 33

### Polymerization of THF with Bis (n-cyclopentadienyl)bis (trifluoromethanesulfonato) titanium and Acetyl Chloride

In a dry box, bis (*n*-cyclopentadienyl)-bis (trifluoromethanesulfonato) titanium (0.50 g) was added to each of three separate oven dried 100 mL RB flasks equipped with stirring bars. The flasks were sealed with rubber septa and then removed from the dry box. Nitrogen bleeds were attached and THF (10 mL) and acetyl chloride (0.375 mL) were added to each flask. After 15, 30, and 45 minutes a polymerization was terminated via the addition of water (25 mL), ether (25 mL) and THF (50 mL). The separated organic phases were washed repeatedly with water (3 x 25 mL), then separated, concentrated at reduced pressure and then dried under vacuum. Polymer yields:

| Polymer. Time | Polymer Yield (%) |
|---|---|
| 15 mins. | 46.11 |
| 30 mins. | 66.85 |
| 45 mins. | 74.97 |

### EXAMPLE 34

### Polymerization of THF with Bis (n-cyclopentadienyl) tetrahydrofuran-bis (trifluoromethanesulfonato) zirconium and Acetic Anhydride

In a dry box, bis (n-cyclopentadienyl) tetrahydrofuran-bis (trifluoromethanesulfonato) zirconium (0.50 g) was added to each of three separate oven dried 100 mL RB flasks equipped with stirring bars. The flasks were sealed with rubber septa and removed from the dry box. After the attachment of nitrogen bleeds THF (20 mL) and acetic anhydride (1.00 mL) were added to each flask. After 15, 30, and 45 minutes, a polymerization was terminated via the addition of water (25 mL), THF (50 mL) and ether (25 mL). The resulting organic phases were separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 15.22 | 11300 | 11900 | 1.05 |
| 30 mins. | 30.50 | 18100 | 20300 | 1.12 |
| 45 mins. | 39.35 | 21300 | 25500 | 1.20 |

### EXAMPLE 35

### Copolymerization of THF and 3-Methyl-THF with Bis (n-cyclopentadienyl) tetrahydrofuran-bis (trifluoromethanesulfonato) zirconium and Acetic Anhydride

In a dry box, bis (*n*-cyclopentadienyl) tetrahydrofuran-bis (trifluoromethanesulfonato) zirconium (0.50 g) was added to each of three separate oven dried 100 mL RB flasks equipped with stirring bars. The flasks were sealed with rubber septa and removed from the dry box. After the attachment of nitrogen bleeds THF (7.5 mL), 3-Methyl-THF (2.5 mL) and acetic anhydride (0.10 mL) were added to each flask. After 15, 30, and 45 minutes, a polymerization was terminated via the addition of water (25 mL), THF (50 mL) and ether (25 mL). The resulting organic phases were separated, concentrated at reduced pressure and then dried under vacuum. ¹H NMR analysis indicates ~10.5% incorporation of 3-methyl-THF in the polymers. Polymer yields and GPC analysis:

| Polymer. Time | Polymer Yield (%) | Mn (PS STD) | Mw | PD |
|---|---|---|---|---|
| 15 mins. | 24.8 | 8500 | 9430 | 1.11 |
| 30 mins. | 41.15 | 11400 | 13300 | 1.17 |
| 45 mins. | 49.15 | 12200 | 15500 | 1.27 |

### EXAMPLE 36

### Preparation of Bis (n-cyclopentadienyl) tetrahydrofuran-bis (trifluoromethanesulfonato) hafnium

In a dry box, hafnocene dichloride (9.93 g) was dissolved in THF (300 mL). To this solution, with stirring, was added a solution of silver triflate (14.12 g) in THF (100 mL). After 10 minutes the precipitated silver chloride was filtered off and the resulting filtrate concentrated to approximately half its volume at reduced pressure. Hexane (250 mL) was added and the resulting mixture placed in the freezer. The resulting precipitate was filtered and then dried under vacuum. Yield: 10.02 g. ¹H NMR (CDCl₃) : 6.68 (s, 10 H), 3.76 (m, 4H), 1.84 (m, 4H).

### EXAMPLE 37

### Preparation of Bis (pentamethyl-n-cyclopentadienyl)-bis (trifluoromethanesulfonato) zirconium

In a dry box, bis (pentamethylcyclopentadienyl)-zirconium dichloride (10.00 g) was dissolved in THF (300 mL). To this solution, with stirring, was added a solution of silver triflate (12.46 g) in THF (100 mL). After 15 minutes the precipitated silver chloride was filtered off and the resulting filtrate concentrated to approximately half its volume at reduced pressure. Hexane (250 mL) was added and the resulting mixture placed in the freezer. The resulting yellow precipitate was filtered and then dried under vacuum. Yield: 6.02 g. ¹H NMR (CDCl₃): 2.12 (s).

### EXAMPLE 38

### Preparation of Bis (n-cyclopentadienyl)-bis (trifluoromethanesulfonato) vanadium

In a dry box, vanadocene dichloride (5.00 g) was dissolved in THF (300 mL). To this solution, with stirring, was added a solution of silver triflate (11.19 g) in THF (100 mL). After 15 minutes the precipitated silver chloride was filtered off and the resulting filtrate concentrated to approximately half its volume at reduced pressure. Hexane (250 mL) was added and the resulting mixture placed in the freezer. The resulting green precipitate was filtered and then dried under vacuum. Yield: 6.99 g.

### EXAMPLE 39

### Polymerization of THF with bis (n-cyclopentadienyl) tetrahydrofuranbis (trifluoromethanesulfonato)zirconium and Acetic Anhydride in Hexane

In a dry box, bis (*n*-cyclopentadienyl) tetrahydrofuran-bis (trifluoromethanesulfonato) zirconium (0.50 g) was added to each of three separate oven dried 100 mL RB flasks equipped with stirring bars. The flasks were sealed with rubber septa and removed from the dry box. After the attachment of nitrogen bleeds hexane (10 mL), THF (20 mL) and acetic anhydride (0.10 mL) were added to each flask. After 15, 30, and 45 minutes, a polymerization was terminated via the addition of water (25 mL), THF (50 mL) and ether (25 mL). The resulting organic phases were separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 4.96 | 1390 | 2020 | 1.45 |
| 30 mins. | 9.24 | 2980 | 3470 | 1.16 |
| 45 mins. | 20.40 | 3410 | 4030 | 1.18 |

### EXAMPLE 40

### Polymerization of Cyclohexene Oxide with bis (n-cyclopentadienyl) tetrahydrofuran-bis (trifluoromethanesulfonato) zirconium

In a dry box, bis (*n*-cyclopentadienyl) tetrahydrofuran-bis (trifluoromethanesulfonato) zirconium (0.50 g) was added to an oven dried 100 mL RB flask equipped with stirring bar, reflux condenser and addition funnel. The flask was sealed with a rubber septum and removed from the dry box. After the attachment of a nitrogen bleed, toluene (10 mL) was added. Then a solution of cyclohexene oxide (20 mL) and toluene (10 mL) was slowly added via the addition funnel. After 60 minutes the polymerization was terminated by the addition of water (25 mL) and toluene (100 mL). The separated organic phase was concentrated at reduced pressure and then dried under vacuum. Polymer yield: 2.28 g. GPC analysis (PS STD.): Mn = 13600, Mw = 24500, PD = 1.80.

### EXAMPLE 41

### Polymerization of THF with Bis (n-cyclopentadienyl) tetrahydrofuran-bis (trifluoromethanesulfonato) hafnium and Acetic Anhydride

In a dry box, bis (*n*-cyclopentadienyl) tetrahydrofuran-bis (trifluoromethanesulfonato) hafnium (0.50 g) was added to each of four separate oven dried 100 mL RB flasks equipped with stirring bars. The flasks were sealed with rubber septa and removed from the dry box. After the attachment of nitrogen bleeds THF (10 mL) and acetic anhydride (0.50 mL) were added to each flask. After 15, 30, 4.5 and 60 minutes, a polymerization was terminated via the addition of water (25 mL), THF (50 mL) and ether (25 mL). The resulting organic phases were separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 32.13 | 11200 | 12200 | 1.09 |
| 30 mins. | 48.70 | 15200 | 18600 | 1.22 |
| 45 mins. | 58.74 | 17400 | 23100 | 1.33 |
| 60 mins. | 60.54 | 17000 | 24100 | 1.42 |

### EXAMPLE 42

### Polymerization of THF with Bis (n-cyclopentadienyl)-bis (trifluoromethanesulfonato) vanadium and Acetic Anhydride

In a dry box, bis (*n*-cyclopentadienyl)-bis (trifluoromethanesulfonato) vanadium (0.50 g) was added to each of four separate oven dried 100 mL RB flasks equipped with stirring bars. The flasks were sealed with rubber septa and removed from the dry box. After the attachment of nitrogen bleeds THF (10 mL) and acetic anhydride (0.50 mL) were added to each flask. After 15, 30, 45 and 60 minutes, a polymerization was terminated via the addition of water (25 mL), THF (50 mL) and ether (25 mL). The resulting organic phases were separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 17.59 | 10600 | 13000 | 1.22 |
| 30 mins. | 45.32 | 14100 | 18800 | 1.34 |
| 45 mins. | 60.43 | 15100 | 21700 | 1.44 |
| 60 mins. | 62.57 | 10500 | 21000 | 2.00 |

### EXAMPLE 43

### Polymerization of THF with Bis (pentamethylcyclopentadienyl) bis (trifluoromethanesulfonato)zirconium and Acetic Anhydride

In a dry box, bis (pentamethylcyclopentadienyl)bis (trifluoromethanesulfonato) zirconium (0.50 g) was added to each of four separate oven dried 100 mL RB flasks equipped with stirring bars. The flasks were sealed with rubber septa and removed from the dry box. After the attachment of nitrogen bleeds THF (10 mL) and acetic anhydride (0.50 mL) were added to each flask. After 15, 30, 45 and 60 minutes, a polymerization was terminated via the addition of water (25 mL), THF (50 mL) and ether (25 mL). The resulting organic phases were separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 33.26 | 10600 | 11900 | 1.12 |
| 30 mins. | 44.64 | 12100 | 14800 | 1.23 |
| 45 mins. | 60.09 | 13400 | 17600 | 1.31 |
| 60 mins. | 70.23 | 15100 | 20900 | 1.38 |

### EXAMPLE 44

### Polymerization of THF with Bis (pentamethylcyclopentadienyl) bis (trifluoromethanesulfonato)zirconium and Adipoyl Chloride

In a dry box, bis (pentamethylcyclopentadienyl)bis (trifluoromethanesulfonato) zirconium (0.50 g) was added to an oven dried 100 mL RB flask equipped with a stirring bar. The flask was sealed with a rubber septum and removed from the dry box. After the attachment of a nitrogen bleed THF (10 mL) and adipoyl chloride (0.50 mL) were added. After 45 minutes, the polymerization was terminated via the addition of water (25 mL), THF (50 mL) and ether (25 mL). The resulting organic phase was separated, concentrated at reduced pressure and then dried under vacuum. Polymer yield: 5.87 g (66.17%).

### EXAMPLE 45

### Polymerization of THF with Bis (pentamethylcyclopentadienyl)bis (trifluoromethanesulfonato)zirconium and Acetyl Bromide

In a dry box, bis (pentamethylcyclopentadienyl)bis (trifluoromethanesulfonato) zirconium (0.50 g) was added to an oven dried 100 mL RB flask equipped with a stirring bar. The flask was sealed with rubber septum and removed from the dry box. After the attachment of a nitrogen bleed THF (10 mL) and acetyl bromide (0.50 mL) were added. After 45 minutes, the polymerization was terminated via the addition of water (25 mL), THF (50 mL) and ether (25 mL). The resulting organic phase was separated, concentrated at reduced pressure and then dried under vacuum. Polymer yield: 2.20 g.

### EXAMPLE 46

### Polymerization of THF with Bis (n-cyclopentadienyl)bis (trifluoromethanesulfonato) vanadium and Acetyl Bromide

In a dry box, bis (*n*-cyclopentadienyl)-bis (trifluoromethanesulfonato) vanadium (0.50 g) was added to an oven dried 100 mL RB flask equipped with a stirring bar. The flask was sealed with rubber septum and removed from the dry box. After the attachment of a nitrogen bleed THF (10 mL) and acetyl bromide (0.50 mL) were added. After 60 minutes, the polymerization was terminated via the addition of water (25 mL), THF (50 mL) and ether (25 mL). The resulting organic phase was separated, concentrated at reduced pressure and then dried under vacuum. Polymer yield: 3.68 g.

### EXAMPLE 47

### Polymerization of THF with Bis (n-cyclopentadienyl) tetrahydrofuran-bis (trifluoromethanesulfonato) hafnium and Acetyl Bromide

In a dry box, bis (n-cyclopentadienyl) tetrahydrofuran-bis (trifluoromethanesulfonato) hafnium (0.50 g) was added to an oven dried 100 mL RB flask equipped with a stirring bar. The flask was sealed with a rubber septum and removed from the dry box. After the attachment of a nitrogen bleed THF (10 mL) and acetyl bromide (0.50 mL) were added. After 30 minutes, the polymerization was terminated via the addition of water (25 mL), THF (50 mL) and ether (25 mL). The resulting organic phase was separated, concentrated at reduced pressure and then dried under vacuum. Polymer yield: 2.29 g.

### EXAMPLE 48

### Polymerization of Oxepane with bis (n-cyclopentadienyl) tetrahydrofuran-bis (trifluoromethanesulfonato) zirconium and Acetic anhydride

In a dry box, bis (*n*-cyclopentadienyl) tetrahydrofuran-bis (trifluoromethanesulfonato) zirconium (0.05 g) was added to an oven dried 50 mL RB flask equipped with stirring bar. The flask was sealed with a rubber septum and removed from the dry box. After the attachment of a nitrogen bleed oxepane (1.00 mL) and acetic anhydride. (0.05 mL) were added via syringe. After 60 minutes the polymerization was terminated by the addition of water (10 mL) and ether (25 mL). The separated organic phase was concentrated at reduced pressure and then dried under vacuum. Polymer yield: 0.87 g.

### EXAMPLE 49

### Polymerization of THF with Bis (n-cyclopentadienyl) tetrahydrofuran-bis (trifluoromethanesulfonato) zirconium and Diglycolyl Chloride

In a dry box, bis (*n*-cyclopentadienyl) tetrahydrofuran-bis (trifluoromethane-sulfonato) zirconium (0.50 g) was added an oven dried 100 mL RB flask equipped with a stirring bar. The flask was sealed with a rubber septum and removed from the dry box. After the attachment of a nitrogen bleed THF (10 mL) and diglycolyl chloride (1.00 mL) were added to the flask. After 45 minutes the polymerization was terminated via the addition of water (25 mL), THF (50 mL) and ether (25 mL). The resulting organic phase was separated, concentrated at reduced pressure and then dried under vacuum. Polymer yield: 0.64 g.

### EXAMPLE 50

### Copolymerization of THF/3-Methyl-THF with Bis (n-cyclopentadienyl) tetrahydrofuran-bis (trifluoromethanesulfonato) zirconium and Diglycolyl Chloride

In a dry box, bis (*n*-cyclopentadienyl) tetrahydrofuran-bis (trifluoromethanesulfonato) zirconium (0.50 g) was added an oven dried 100 mL RB flask equipped with a stirring bar. The flask was sealed with a rubber septum and removed from the dry box. After the attachment of a nitrogen bleed THF (7.5 mL), 3-methyl-THF (2.5 mL) and diglycolyl chloride (1.00 mL) were added to the flask. After 45 minutes the polymerization was terminated via the addition of water (25 mL), THF (50 mL) and ether (25 mL). The resulting organic phase was separated, concentrated at reduced pressure and then dried under vacuum. Polymer yield: 0.63 g.

### EXAMPLE 51

### Polymerization of THF with Bis (pentamethylcyclopentadienyl)-bis (trifluoromethanesulfonato) zirconium and Trifluoroacetic Anhydride

In a dry box, bis (pentamethylcyclopentadienyl)bis (trifluoromethanesulfonato) zirconium (0.50 g) was added to an oven dried 100 mL RB flask equipped with a stirring bar. The flask was sealed with a rubber septum and removed from the dry box. After the attachment of a nitrogen bleed THF (10 mL) and trifluoroactic anhydride (0.50 mL) were added. After 3 hrs., the polymerization was terminated via the addition of water (25 mL), THF (50 mL) and ether (25 mL). The resulting organic phase was separated, concentrated at reduced pressure and then dried under vacuum. Polymer yield: 4.89 g.

### EXAMPLE 52

### Copolymerization of THF/3-Methyl-THF with Bis (n-cyclopentadienyl) tetrahydrofuran-bis (trifluoromethanesulfonato) zirconium and Adipoyl Chloride

In a dry box, bis (*n*-cyclopentadienyl) tetrahydrofuran-bis (trifluoromethanesulfonato) zirconium (0.50 g) was added an oven dried 100 mL RB flask equipped with a stirring bar. The flask was sealed with a rubber septum and removed from the dry box. After the attachment of a nitrogen bleed THF (7.5 mL), 3-methyl-THF (2.5 mL) and adipoyl chloride (1.00 mL) were added to the flask. After 60 minutes the polymerization was terminated via the addition of water (25 mL), THF (50 mL) and ether (25 mL). The resulting organic phase was separated, concentrated at reduced pressure and then dried under vacuum. Polymer yield: 5.98 g.

### EXAMPLE 53

### Polymerization of THF with Yttrium Triflate and Acetyl Chloride

In a dry box, yttrium triflate (0.75 g) was added to each of four separate oven dried 100 mL RB flasks equipped with stirring bars. The flasks were sealed with rubber septa and then removed from the dry box.
Nitrogen bleeds were attached and THF (20 mL) and acetyl chloride (0.75 mL) were added to each flask. After 15, 30, 45 and 60 minutes a polymerization was terminated via the addition of water (25 mL), ether (25 mL) and THF (50 mL). The resulting organic phases were separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 49.21 | 1610 | 3470 | 2.15 |
| 30 mins. | 50.05 | 1520 | 3390 | 2.22 |
| 45 mins. | 49.77 | 1510 | 3570 | 2.36 |
| 60 mins. | 52.76 | 1740 | 3940 | 2.26 |

### EXAMPLE 54

### Polymerization of THF with Ytterbium Triflate and Acetyl Chloride

In a dry box, ytterbium triflate (0.75 g) was added to each of three separate oven dried 100 mL RB flasks equipped with stirring bars. The flasks were sealed with rubber septa and then removed from the dry box. Nitrogen bleeds were attached and THF (20 mL) and acetyl chloride (0.75 mL) were added to each flask. After 15, 30 and 45 minutes a polymerization was terminated via the addition of water (25 mL), ether (25 mL) and THF (50 mL). The resulting organic phases were separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 52.59 | 1710 | 3790 | 2.22 |
| 30 mins. | 52.82 | 1730 | 4540 | 2.61 |
| 45 mins. | 52.25 | 1730 | 4690 | 2.71 |

### EXAMPLE 55

### Polymerization of THF with Didymium (Mischmetall) Triflate and Acetyl Chloride

In a dry box, didymium triflate (0.75 g) was added to each of three separate oven dried 100 mL RB flasks equipped with stirring bars. The flasks were sealed with rubber septa and then removed from the dry box. Nitrogen bleeds were attached and THF (20 mL) and acetyl chloride (0.75 mL) were added to each flask. After 15, 30 and 45 minutes a polymerization was terminated via the addition of water (25 mL), ether (25 mL) and THF (50 mL). The resulting organic phases were separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 21.98 | 1020 | 2000 | 1.95 |
| 30 mins. | 26.94 | 926 | 1780 | 1.92 |
| 45 mins. | 32.86 | 1040 | 2060 | 1.97 |

### EXAMPLE 56

### Polymerization of THF with Erbium Triflate and Acetyl Chloride

In a dry box, erbium triflate (0.75 g) was added to each of three separate oven dried 100 mL RB flasks equipped with stirring bars. The flasks were sealed with rubber septa and then removed from the dry box. Nitrogen bleeds were attached and THF (20 mL) and acetyl chloride (0.75 mL) were added to each flask. After 15, 30 and 45 minutes a polymerization was terminated via the addition of water (25 mL), ether (25 mL) and THF (50 mL). The resulting organic phases were separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 53.83 | 1570 | 3400 | 2.17 |
| 30 mins. | 56.09 | 1650 | 4090 | 2.47 |
| 45 mins. | 56.99 | 1710 | 4310 | 2.51 |

### EXAMPLE 57

### Polymerization of THF with Scandium Triflate and Acetyl Chloride

In a dry box, scandium triflate (0.75 g) was added to each of three separate oven dried 100 mL RB flasks equipped with stirring bars. The flasks were sealed with rubber septa and then removed from the dry box. Nitrogen bleeds were attached and THF (20 mL) and acetyl chloride (0.75 mL) were added to each flask. After 15, 30 and 45 minutes a polymerization was terminated via the addition of water (25 mL), ether (25 mL) and THF (50 mL). The resulting organic phases were separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 53.33 | 1750 | 4180 | 2.38 |
| 30 mins. | 54.17 | 1690 | 4630 | 2.73 |
| 45 mins. | 53.49 | 1570 | 5660 | 3.61 |

### EXAMPLE 58

### Polymerization of THF with Copper Triflate and Acetyl Chloride

In a dry box, copper triflate (0.75 g) was added to each of three separate oven dried 100 mL RB flasks equipped with stirring bars. The flasks were sealed with rubber septa and then removed from the dry box.
Nitrogen bleeds were attached and THF (20 mL) and acetyl chloride (0.75 mL) were added to each flask. After 15, 30 and 45 minutes a polymerization was terminated via the addition of water (25 mL), ether (25 mL) and THF (50 mL). The resulting organic phases were separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 23.56 | 1010 | 2150 | 2.13 |
| 30 mins. | 31.74 | 1250 | 2720 | 2.18 |
| 45 mins. | 43.24 | 1390 | 3180 | 2.29 |

### EXAMPLE 59

### Polymerization of THF with Tin Triflate and Acetyl Chloride

In a dry box, tin triflate (0.75 g) was added to each of three separate oven dried 100 mL RB flasks equipped with stirring bars. The flasks were sealed with rubber septa and then removed from the dry box. Nitrogen bleeds were attached and THF (20 mL) and acetyl chloride (0.75 mL) were added to each flask. After 15, 30 and 45 minutes a polymerization was terminated via the addition of water (25 mL), ether (25 mL) and THF (50 mL). The resulting organic phases were separated, concentrated at reduced pressure and then dried under vacuum. Polymer yields:

| Polymer. Time | Polymer Yield (%) |
|---|---|
| 15 mins. | 23.96 |
| 30 mins. | 40.53 |
| 45 mins. | 41.60 |

### EXAMPLE 60

### Polymerization of THF with Zirconium Triflate and Acetyl Chloride

In a dry box, zirconium triflate (0.75 g) was added to each of three separate oven dried 100 mL RB flasks equipped with stirring bars. The flasks were sealed with rubber septa and then removed from the dry box.
Nitrogen bleeds were attached and THF (20 mL) and acetyl chloride (0.75 mL) were added to each flask. After 15, 30 and 45 minutes a polymerization was terminated via the addition of water (25 mL), ether (25 mL) and THF (50 mL). The resulting organic phases were separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 49.04 | 2040 | 4320 | 2.12 |
| 30 mins. | 64.43 | 2200 | 4880 | 2.21 |
| 45 mins. | 65.84 | 2290 | 5190 | 2.27 |

### EXAMPLE 61

### Polymerization of THF with Zinc Triflate and Acetyl Chloride

In a dry box, zinc triflate (0.75 g) was added to each of three separate oven dried 100 mL RB flasks equipped with stirring bars. The flasks were sealed with rubber septa and-then removed from the dry box. Nitrogen bleeds were attached and THF (20 mL) and acetyl chloride (0.75 mL) were added to each flask. After 45, 60 and 75 minutes a polymerization was terminated via the addition of water (25 mL), ether (25 mL) and THF (50 mL). The resulting organic phases were separated, concentrated at reduced pressure and then dried under vacuum. Polymer yields:

| Polymer. Time | Polymer Yield (%) |
|---|---|
| 15 mins. | 5.64 |
| 30 mins. | 6.88 |
| 45 mins. | 7.61 |

### EXAMPLE 62

### Polymerization of THF with Yttrium Triflate and Adipoyl Chloride

In a dry box, yttrium triflate (1.00 g) was added to each of three separate oven dried 100 mL RB flasks equipped with stirring bars. The flasks were sealed with rubber septa and then removed from the dry box. Nitrogen bleeds were attached and THF (20 mL) and adipoly chloride (1.00 mL) were added to each flask. After 15, 30 and 45 minutes a polymerization was terminated via the addition of water (25 mL), ether (25 mL) and THF (50 mL). The resulting organic phases were separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 56.20 | 2020 | 4350 | 2.16 |
| 30 mins. | 58.62 | 2350 | 4790 | 2.04 |
| 45 mins. | 58.40 | 1910 | 5250 | 2.75 |

### EXAMPLE 63

### Polymerization of THF with Terephthaloyl Chloride and Yttrium Triflate

In a dry box, yttrium triflate (0.75 g) and terephthaloyl chloride (2.00 g) were added to a 100 mL RB flask equipped with a stiiring bar. The flask was sealed with a rubber septum and removed from the dry box. A nitrogen purge was attached and THF (20 mL) addeded via syringe. After 90 minutes the polymerization was terminated by the addition of water (25 mL) and THF (25 mL) and ether (50 mL). The separated organic phase was concentrated at reduced pressure and then dried under vacuum. Polymer yield: 2.25 g. GPC Analysis (PS STD.): Mn = 40900, Mw = 63000, PD = 1.54.

### EXAMPLE 64

### Polymerization of THF with Neodymium Triflate and Acetyl Bromide

In a dry box, neodymium triflate (0.75 g) was added to each of four separate oven dried 100 mL RB flasks equipped with stirring bars. The flasks were sealed with rubber septa and then removed from the dry box.
Nitrogen bleeds were attached and THF (20 mL) and acetyl bromide (1.50 mL) were added to each flask. After 15, 30, 45 and 60 minutes a polymerization was terminated via the addition of water (25 mL), ether (25 mL) and THF (50 mL). The resulting organic phases were separated, concentrated at reduced pressure and then dried under vacuum. Polymer yields:

| Polymer. Time | Polymer Yield (%) |
|---|---|
| 15 mins. | 27.11 |
| 30 mins. | 27.06 |
| 45 mins. | 28.13 |
| 60 mins. | 27.28 |

### EXAMPLE 65

### Polymerization of THF with Diglycolyl Chloride and Ytterbium Triflate

In a dry box, ytterbium triflate (1.00 g) was added to a 100 mL RB flask equipped with a stirring bar. The flask was sealed with a rubber septum and removed from the dry box. A nitrogen purge was attached and THF (20 mL) addeded via syringe, followed by diglycolyl chloride (2.00 mL, 97%). After 60 minutes the polymerization was terminated by the addition of water (25 mL) and THF (25 mL) and ether (50 mL). The separated organic phase was concentrated at reduced pressure and then dried under vacuum. Polymer yield: 9.53 g.

### EXAMPLE 66

### Polymerization of THF with Diglycolyl Chloride and Zirconium Triflate

In a dry box, zirconium triflate (1.00 g) was added to a 100 mL RB flask equipped with a stiiring bar. The flask was sealed with a rubber septum and removed from the dry box. A nitrogen purge was attached and THF (20 mL) added via syringe, followed by diglycolyl chloride (2.00 mL, 97%). After 60 minutes the polymerization was terminated by the addition of water (25 mL) and THF (25 mL) and ether (50 mL). The separated organic phase was concentrated at reduced pressure and then dried under vacuum. Polymer yield: 7.32 g.

### EXAMPLE 67

### Copolymerization of THF/3-Methyl-THF with Ytterbium Triflate and Adipoyl Chloride

In a dry box, ytterbium triflate (0.50 g) was added an oven dried 100 mL RB flask equipped with a stirring bar. The flask was sealed with a rubber septum and removed from the dry box. After the attachment of a nitrogen bleed THF (7.5 mL), 3-methyl-THF (2.5 mL) and adipoyl chloride (1.00 mL) were added to the flask. After 60 minutes the polymerization was terminated via the addition of water (25 mL), THF (50 mL) and ether (25 mL). The resulting organic phase was separated, concentrated at reduced pressure and then dried under vacuum. Polymer yield: 5.2 g.

### EXAMPLE 68

### Polymerization of THF with Scandium Triflate and Acetic Anhydride

In a dry box, scandium triflate (0.75 g) was added to each of four separate oven dried 100 mL RB flasks equipped with stirring bars. The flasks were sealed with rubber septa and removed from the dry box. After the attachment of nitrogen bleeds THF (20 mL) and acetic anhydride (0.75 mL) were added to each flask. After 15, 30, 45 and 60 minutes, a polymerization was terminated via the addition of water (25 mL) and THF (50 mL). The resulting organic phases were separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 15 mins. | 63.64 | 3780 | 11000 | 2.91 |
| 30 mins. | 70.85 | 3270 | 9270 | 2.82 |
| 45 mins. | 70.85 | 2780 | 9740 | 3.49 |
| 60 mins. | 74.18 | 2930 | 8330 | 2.84 |

### EXAMPLE 69

### Polymerization of THF with Copper Triflate and Acetic Anhydride

In a dry box, copper triflate (0.75 g) was added to each of four separate oven dried 100 mL RB flasks equipped with stirring bars. The flasks were sealed with rubber septa and then removed from the dry box.
Nitrogen bleeds were attached and THF (20 mL) and acetic anhydride (0.75 mL) were added to each flask. After 45, 60, 75 and 90 minutes a polymerization was terminated via the addition of water (25 mL) and THF (50 mL). The separated organic phases were separated, concentrated at reduced pressure and then dried under vacuum. Polymer yields and GPC analysis:

| Polymer. Time | Polymer Yield (%) | Mn (PS STD) | Mw | PD |
|---|---|---|---|---|
| 45 mins. | 23.90 | 10500 | 21100 | 2.01 |
| 60 mins. | 30.10 | 12000 | 23400 | 1.95 |
| 75 mins. | 35.00 | 11400 | 23500 | 2.07 |
| 90 mins. | 53.21 | 13900 | 25900 | 1.86 |

### EXAMPLE 70

### polymerization of THF with Zirconium Triflate and Acetic anhydride

In a dry box, zirconium triflate (0.75 g) was added to each of four separate oven dried 100 mL RB flasks equipped with stirring bars. The flasks were sealed with rubber septa and removed from the dry box.
Nitrogen bleeds were attached and THF (20 mL) and acetic anhydride (0.75 mL) were added to each flask. After 15, 30, 45 and 60 minutes, a polymerization was terminated via the addition of water (25 mL) and THF (50 mL). The resulting organic phases were separated, concentrated at reduced pressure and then dried under vacuum. Polymer yields:

| Polymer. Time | Polymer Yield (%) |
|---|---|
| 15 mins. | 58.06 |
| 30 mins. | 65.84 |
| 45 mins. | 66.91 |
| 60 mins. | 71.87 |

### EXAMPLE 71

### Polymerization of THF with Tin Triflate and Acetic Anhydride

In a dry box, tin triflate (0.75 g) was added to each of four separate oven dried 100 mL RB flasks equipped with stirring bars. The flasks were sealed with rubber septa and removed from the dry box.
Nitrogen bleeds were attached and THF (20 mL) and acetic anhydride (0.75 mL) were added to each flask. After 15, 30, 45 and 90 minutes, a polymerization was terminated via the addition of water (25 mL) and THF (50 mL). The resulting organic phases were separated, concentrated at reduced pressure and then dried under vacuum. Polymer yields:

| Polymer. Time | Polymer Yield (%) |
|---|---|
| 15 mins. | 24.01 |
| 30 mins. | 44.08 |
| 45 mins. | 54.68 |
| 60 mins. | 58.40 |

### EXAMPLE 72

### Polymerization of THF with Zinc Triflate and Acetic Anhydride

In a dry box, zinc triflate (0.75 g) was added to an oven dried 100 mL RB flask equipped with a stirring bar. The flask was sealed with a rubber septum and removed from the dry box. A nitrogen bleed was attached and THF (20 mL) and acetic anhydride (0.75 mL) were added. After stirring overnight the polymerization was terminated via the addition of water (25 mL) and THF (50 mL). The resulting organic phase was separated, concentrated at reduced pressure and then dried under vacuum. Polymer Yield: 3.17 g (17.87%).

### EXAMPLE 73

### Copolymerization of THF/3-Methyl-THF with Zirconium Triflate and Acetic Anhydride

In a dry box, zirconium triflate (0.50 g) was added to an oven dried 100 mL RB flask equipped with a stirring bar. The flask was sealed with a rubber septum and removed from the dry box. A nitrogen bleed was attached and THF (7.5 mL) and 3-methyl-THF (2.5 mL) were added followed by acetic anhydride (1.00 mL). After 45 minutes the polymerization was terminated via the addition of water (25 mL) and THF (50 mL). The resulting organic phase was separated, concentrated at reduced pressure and then dried under vacuum. Polymer yield: 5.68 g.

### EXAMPLE 74

### Copolymerization of THF/3-Methyl-THF with Copper Triflate and Acetic Anhydride

In a dry box, copper triflate (0.50 g) was added to an oven dried 100 mL RB flask equipped with a stirring bar. The flask was sealed with a rubber septum and removed from the dry box. A nitrogen bleed was attached and THF (7.5 mL) and 3-methyl-THF (2.5 mL) were added followed by acetic anhydride (1.00 mL). After 60 minutes the polymerization was terminated via the addition of water (25 mL) and THF (50 mL). The resulting organic phase was separated, concentrated at reduced pressure and then dried under vacuum. Polymer yield: 2.48 g.

### EXAMPLE 75

### Copolymerization of THF/3-Methyl-THF with Tin Triflate and Acetic Anhydride

In a dry box, tin triflate (0.50 g) was added to an oven dried 100 mL RB flask equipped with a stirring bar. The flask was sealed with a rubber septum and removed from the dry box. A nitrogen bleed was attached and THF (7.5 mL) and 3-methyl-THF (1.6 mL) were added followed by acetic anhydride (1.00 mL). After 60 minutes the polymerization was terminated via the addition of water (25 mL) and THF (50 mL). The resulting organic phase was separated, concentrated at reduced pressure and then dried under vacuum. Polymer yield: 4.42 g.

### EXAMPLE 76

### Copolymerization of THF/3-Methyl-THF with Scandium Triflate and Acetic Anhydride

In a dry box, scandium triflate (0.50 g) was added to an oven dried 100 mL RB flask equipped with a stirring bar. The flask was sealed with a rubber septum and removed from the dry box. A nitrogen bleed was attached and THF (7.5 mL) and 3-methyl-THF (2.5 mL) were added followed by acetic anhydride (1.00 mL). After 45 minutes the polymerization was terminated via the addition of water (25 mL) and THF (50 mL). The resulting organic phase was separated, concentrated at reduced pressure and then dried under vacuum. Polymer yield: 5.81 g.

### EXAMPLE 77

### Polymerization of THF with Copper Triflate and Trifluoroacetic Anhydride

In a dry box, copper triflate (1.00 g) was added to an oven dried 100 mL RB flask equipped with a stirring bar. The flask was sealed with a rubber septum and removed from the dry box. A nitrogen bleed was attached and THF (20 mL) and trifluoroacetic anhydride (2.00 mL) were added. After stirring for 3 hrs. the polymerization was terminated via the addition of water (25 mL) and THF (50 mL). The resulting organic phase was separated, concentrated at reduced pressure and then dried under vacuum. Polymer Yield: 7.5 g.

### EXAMPLE 78

### Polymerization of THF with Trifluoroacetic Acid and Ytterbium Triflate at 45°C

In a dry box, ytterbium triflate (5.00 g) was added to an oven dried 100 mL RB flask equipped with a stirring bar. The flask was sealed with rubber septa and removed from the dry box. After the attachment of nitrogen bleeds THF (20.00 mL) and trifluoroacetic acid (4.00 mL) were added to the flask. Then flask was them immediately placed in an oil bath maintained at 45°C. After 120 minutes the polymerization was terminated via the addition of water (25 mL), THF (50 mL) and ether (25 mL). The resulting organic phase was separated, concentrated at reduced pressure and then dried under vacuum. Polymer yield: 6.61 g. GPC analysis (PS STD.): Mn = 5680, Mw = 9090, PD = 1.60.

### EXAMPLE 79

### Polymerization of THF with Trifluoroacetic Acid and Ytterbium Triflate at 45°C

In a dry box, ytterbium triflate (5.00 g) was added to an oven dried 100 mL RB flask equipped with a stirring bar. The flask was sealed with rubber septa and removed from the dry box. After the attachment of a nitrogen bleed THF (20.00 mL) and trifluoroacetic acid (5.00 mL) were added to the flask. The flask was then immediately placed in an oil bath maintained at 45°C. After 120 minutes the polymerization was terminated via the addition of water (25 mL), THF (50 mL) and ether (25 mL). The resulting organic phase was separated, concentrated at reduced pressure and then dried under vacuum. Polymer yield: 3.07 g. GPC analysis (PS STD.): Mn = 3290, Mw = 4810, PD = 1.46.

### EXAMPLE 80

### Polymerization of THF with Trifluoroacetic Acid and Ytterbium Triflate

In a dry box, ytterbium triflate (2.00, 3.00, 4.00 and 5.00 g) was added to each of four separate oven dried 100 mL RB flasks equipped with stirring bars. The flasks were sealed with rubber septa and removed from the dry box. After the attachment of nitrogen bleeds THF (20.00 mL) and trifluoroacetic acid (2.00 mL) were added to each flask. After 90 minutes the polymerizations were terminated via the addition of water (25 mL), THF (50 mL) and ether (25 mL). The resulting organic phases were separated, concentrated at reduced pressure and then dried under vacuum. Polymer yields and GPC analyses:

| Ytterbium Triflate (g) | Polymer Yield (g) | Mn (PS STD.) | Mw | PD |
|---|---|---|---|---|
| 2.00 | 5.32 | 60200 | 95600 | 1.59 |
| 3.00 | 5.95 | 58500 | 89400 | 1.53 |
| 4.00 | 6.70 | 46100 | 76700 | 1.66 |

### EXAMPLE 81

### Polymerization of THF with Trifluoroacetic Acid and Yttrium Triflate

In a dry box, yttrium triflate (3.00 g) was added to each of three separate oven dried 100 mL RB flasks equipped with stirring bars. The flasks were sealed with rubber septa and removed from the dry box. After the attachment of nitrogen bleeds THF (20.00 mL) and trifluoroacetic acid (5.00 mL) were added to each flask. After 120, 150 and 180 minutes a polymerization was terminated via the addition of water (25 mL), THF (50 mL) and ether (25 mL). The resulting organic phases were separated, concentrated at reduced pressure and then dried under vacuum. Polymer yields and GPC analyses:

| Polymer. Time | Polymer Yield (g) | Mn (PS STD.) | Mw | PD |
|---|---|---|---|---|
| 120 mins. | 1.57 | 22700 | 32900 | 1.45 |
| 150 mins. | 2.75 | 24600 | 37900 | 1.54 |
| 180 mins. | 3.69 | 30300 | 46400 | 1.54 |

### EXAMPLE 82

### Polymerization of THF with Trifluoroacetic Acid and Erbium Triflate

In a dry box, erbium triflate (4.00 g) was added to each of five separate oven dried 100 mL RB flasks equipped with stirring bars. The flasks were sealed with rubber septa and removed from the dry box. After the attachment of nitrogen bleeds THF (20.00 mL) and trifluoroacetic acid (5.00 mL) were added to each flask. After 60, 90, 120, 150 and 180 minutes a polymerization was terminated via the addition of water (25 mL), THF (50 mL) and ether (25 mL). The resulting organic phases were separated, concentrated at reduced pressure and then dried under vacuum. Polymer yields and GPC analyses:

| Polymer. Time | Polymer Yield (g) | Mn (PD STD.) | Mw | PD |
|---|---|---|---|---|
| 60 mins. | 2.02 | 13300 | 20900 | 1.57 |
| 90 mins. | 3.13 | 26500 | 36900 | 1.39 |
| 120 mins. | 4.84 | 26600 | 39700 | 1.49 |
| 150 mins. | 5.08 | 30600 | 49600 | 1.62 |
| 180 mins. | 5.58 | 27900 | 45700 | 1.63 |

### EXAMPLE 83

### Copolymerization of THF/3-Methyl-THF with Trifluoroacetic Acid and Ytterbium Triflate

In a dry box, ytterbium triflate (5.00 g) was added to separate oven dried 100 mL RB flasks equipped with stirring bars. The flasks were sealed with rubber septa and removed from the dry box. After the attachment of nitrogen bleeds THF (15.00 mL and 3-methyl-THF (5.00 mL) were added to each flask. Trifluoroacetic acid (3 and 4 mL) was then added to each flask. After 120 minutes the polymerizations were terminated via the addition of water (25 mL), THF (50 mL) and ether (25 mL). The resulting organic phases were separated, concentrated at reduced pressure and then dried under vacuum. Polymer yields and GPC analyses:

| Trifluoroacetic Acid | Polymer Yield (g) | Mn (PS STD.) | Mw | PD |
|---|---|---|---|---|
| 3 mL | 5.37 | 24500 | 37500 | 1.53 |
| 4 mL | 3.9 | 20900 | 30300 | 1.45 |

### EXAMPLE 84

### Polymerization of THF with Trifluoroacetic Anhydride/ Trifluoroacetic Acid and Ytterhium Triflate

In a dry box, ytterbium triflate (3.00 g) was added to each of four separate oven dried 100 mL RB flasks equipped with stirring bars. The flasks were sealed with rubber septa and removed from the dry box. After the attachment of nitrogen bleeds THF (20.00 mL) was added to each flask. Trifluoracetic anhydride and trifluoroacetic acid were added together via syringes in the ratios shown below. After 60 minutes the polymerizations were terminated via the addition of water (25 mL), THF (50 mL) and ether (25 mL). The resulting organic phases were separated, concentrated at reduced pressure and then dried under vacuum. Polymer yields and GPC analyses:

| Trifluoroacetic Anhydride/ Trifluoroacetic Acid (mL) | Polymer Yield (g) | Mn (PS STD.) | Mw | PD |
|---|---|---|---|---|
| 5/2 | 10.66 | 8090 | 13400 | 1.66 |
| 5/3 | 9.21 | 6600 | 10100 | 1.54 |
| 5/4 | 7.13 | 5200 | 8150 | 1.57 |
| 5/5 | 4.86 | 4200 | 59100 | 1.41 |

### EXAMPLE 85

### Polymerization of THF with Trifluoroacetic Anhydride/Trifluoroacetic Acid and Ytterbium Triflate

In a dry box, ytterbium triflate (3.00 g) was added to an oven dried 100 mL RB flask equipped with a stirring bar. The flask was sealed with a rubber septum and removed from the dry box. After the attachment of a nitrogen bleed THF (20.00 mL) was added to the flask. Trifluoracetic anhydride (3.00 mL) and trifluoroacetic acid (5.00 mL) were added together via syringe. After 60 minutes the polymerization was terminated via the addition of water (25 mL), THF (50 mL) and ether (25 mL). The resulting organic phase was separated, concentrated at reduced pressure and then dried under vacuum. Polymer yield: 6.85 g. GPC analysis: Mn = 5910, Mw = 9970, PD = 1.50 (PS STD.).

### EXAMPLE 86

### Polymerization of THF with Pentafluoropropionic Acid and Ytterbium Triflate

In a dry box, ytterbium triflate (5.00 g) was added to an oven dried 100 mL RB flask equipped with a stirring bar. The flask was sealed with a rubber septum and removed from the dry box. After the attachment of a nitrogen bleed THF (20.00 mL) and pentafluoropropionic acid (2.00 mL) were added via syringe. After 150 minutes the polymerization was terminated via the addition of water (25 mL), THF (50 mL) and ether (25 mL). The resulting organic phase was separated, concentrated at reduced pressure and then dried under vacuum. Polymer yield: 9.42 g. GPC analysis: Mn = 71500, Mw = 126000, PD = 1.77 (PS STD.).

### EXAMPLE 87

### Polymerization of THF with Pentafluoropropionic Acid and Ytterbium Triflate

In a dry box, ytterbium triflate (5.00 g) was added to an oven dried 100 mL RB flask equipped with a stirring bar. The flask was sealed with a rubber septum and removed from the dry box. After the attachment of a nitrogen bleed THF (20.00 mL) and pentafluoropropionic acid (5.00 mL) were added via syringe. After 150 minutes the polymerization was terminated via the addition of water (25 mL), THF (50 mL) and ether (25 mL). The resulting organic phase was separated, concentrated at reduced pressure and then dried under vacuum. Polymer yield: 7.00 g. GPC analysis: Mn = 20100, Mw = 38700, PD = 1.92 (PS STD.).

### EXAMPLE 88

### Polymerization of THF with Cyanoacetic Acid and Ytterbium Triflate

In a dry box, ytterbium triflate (5.00 g) and cyanoacetic acid (5.00 g) were added to an oven dried 100 mL RB flask equipped with a stirring bar. The flask was sealed with a rubber septum and removed from the dry box. After the attachment of a nitrogen bleed THF (20.00 mL) was added via syringe. After 150 minutes the polymerization was terminated via the addition of water (25 mL), THF (50 mL) and ether (25 mL). The resulting organic phase was separated, concentrated at reduced pressure and then dried under vacuum. Polymer yield: 6.15 g. GPC analysis: Mn = 22900, Mw = 33900, PD = 1.48 (PS STD.).

### EXAMPLE 89

### Polymerization of THF with Trifluoroacetic Acid and Zirconium Triflate

In a dry box, zirconium triflate (1.5 g) was added to an oven dried 100 mL RB flask equipped with a stirring bar. The flask was sealed with a rubber septum and removed from the dry box. After the attachment of a nitrogen bleed THF (10 mL) and trifluoroacetic acid (1.5 mL) were added via syringe. After 120 minutes the polymerization was terminated via the addition of water (25 mL), THF (50 mL) and ether (25 mL). The resulting organic phase was separated, concentrated at reduced pressure and then dried under vacuum. Polymer yield: 5.63 g. GPC analysis: Mn = 33300, Mw = 52600, PD = 1.58 (PS STD.).

### EXAMPLE 90

### Polymerization of THF with Chlorodifluoroacetic Acid and Zirconium Triflate

In a dry box, zirconium triflate (2.5 g) was added to an oven dried 100 mL RB flask equipped with a stirring bar. The flask was sealed with a rubber septum and removed from the dry box. After the attachment of a nitrogen bleed THF (10.00 mL) and chlorodifluoroacetic acid (2.50 mL) were added via syringe. After 120 minutes the polymerization was terminated via the addition of water (25 mL), THF (50 mL) and ether (25 mL). The resulting organic phase was separated, concentrated at reduced pressure and then dried under vacuum. Polymer yield: 5.55 g. GPC analysis: Mn = 19600, Mw = 35300, PD = 1.80 (PS STD.).

### EXAMPLE 91

### Polymerization of THF with Trifluoroacetic Acid and Yttrium Triflate

In a dry box, yttrium triflate (3.00 g) was added to an oven dried 100 mL RB flask equipped with a stirring bar. The flask was sealed with a rubber septum and removed from the dry box. After the attachment of a nitrogen bleed THF (20.00 mL) and trifluoroacetic acid (5.00 mL) were added via syringe. After 180 minutes the polymerization was terminated via the addition of water (25 mL), THF (50 mL) and ether (25 mL). The resulting organic phase was separated, concentrated at reduced pressure and then dried under vacuum. Polymer yield: 3.69 g. GPC analysis: Mn = 30300, Mw = 46400, PD = 1.54 (PS STD.).

### EXAMPLE 92

### Copolymerization of THF/3-Methyl-THF with Trifluoroacetic Acid and Ytterbium Triflate

In a dry box, ytterbium triflate (5.00 g) was added to an oven dried 100 mL RB flask equipped with a stirring bar. The flask was sealed with a rubber septum and removed from the dry box. After the attachment of a nitrogen bleed THF (15.00 mL), 3-methyl-THF (5.00 mL) and trifluoroacetic acid (3.00 mL) were added via syringe. After 120 minutes the polymerization was terminated via the addition of water (25 mL), THF (50 mL) and ether (25 mL). The resulting organic phase was separated, concentrated at reduced pressure and then dried under vacuum. Polymer yield: 5.37 g. GPC analysis: Mn = 24500, Mw = 37500, PD = 1.53 (PS STD.).

### EXAMPLE 93

### Polymerization of THF with Acetic Acid and Ytterbium Triflate

In a dry box, ytterbium triflate (5.00 g) was added to each of four separate oven dried 100 mL RB flasks equipped with stirring bars. The flasks were sealed with rubber septa and removed from the dry box. After the attachment of nitrogen bleeds THF (20.00 mL) and acetic acid (5.00 mL) were added to each flask. After 3, 4, 5 and 24 hours a polymerization was terminated via the addition of water (25 mL), THF (50 mL) and ether (25 mL). The resulting organic phases were separated, washed with water (2 x 50 mL), concentrated at reduced pressure and then dried under vacuum. Polymer yields and GPC analyses:

| Polymer. Time (Hrs.) | Polymer Yield (g) | Mn (PS STD.) | Mw | PD |
|---|---|---|---|---|
| 3 | 1.15 | 17900 | 34600 | 1.93 |
| 4 | 1.38 | 18400 | 33700 | 1.83 |
| 5 | 1.71 | 16400 | 34000 | 2.07 |
| 24 | 5.29 | 13000 | 30400 | 2.33 |

### EXAMPLE 94

### Polymerization of THF with Formic Acid (96%) Acid and Ytterbium Triflate

In a dry box, ytterbium triflate (5.00 g) was added to each of six separate oven dried 100 mL RB flasks equipped with stirring bars. The flasks were sealed with rubber septa and removed from the dry box. After the attachment of nitrogen bleeds THF (20.00 mL) and formic acid (96%, 0.75 mL) were added to each flask. After 2, 3, 4, 5, 6 and 24 hours a polymerization was terminated via the addition of water (25 mL), THF (50 mL) and ether (25 mL). The resulting organic phases were separated, washed with water (2 x 50 mL), concentrated at reduced pressure and then dried under vacuum. Polymer yields and GPC analyses:

| Polymer. Time (Hrs.) | Polymer Yield (g) | Mn (PS STD.) | Mw | PD |
|---|---|---|---|---|
| 2 | 1.25 | 14300 | 29500 | 2.06 |
| 3 | 1.65 | 15100 | 30300 | 2.00 |
| 4 | 2.27 | 17600 | 32700 | 1.88 |
| 5 | 2.72 | 16700 | 30800 | 1.85 |
| 6 | 3.29 | 15300 | 29800 | 1.95 |
| 24 | 7.93 | 10700 | 23100 | 2.16 |

### EXAMPLE 95

### Polymerization of THF with Formic Acid (96%) Acid and Ytterbium Triflate

In a dry box, ytterbium triflate (15.77 g) was added to each of six separate oven dried 100 mL RB flasks equipped with stirring bars. The flasks were sealed with rubber septa and removed from the dry box. After the attachment of nitrogen bleeds THF (20.00 mL) and formic acid (96%, 2.00 mL) were added to each flask. After 2, 3, 4, 5, 6 and 24 hours a polymerization was terminated via the addition of water (25 mL), THF (50 mL) and ether (25 mL). The resulting organic phases were separated, washed with water (2 x 50 mL), concentrated at reduced pressure and then dried under vacuum. Polymer yields and GPC analyses:

| Polymer. Time (Hrs.) | Polymer Yield (g) | Mn (PS STD.) | Mw | PD |
|---|---|---|---|---|
| 2 | 1.67 | 4350 | 10900 | 2.51 |
| 3 | 2.22 | 5560 | 12000 | 2.16 |
| 4 | 2.83 | 5320 | 12400 | 2.34 |
| 5 | 3.09 | 5460 | 12100 | 2.22 |
| 6 | 3.28 | 5390 | 11700 | 2.19 |
| 24 | 5.82 | 3050 | 7860 | 2.58 |

### EXAMPLE 96

### Polymerization of THF with Pyruvonitrile and Ytterbium Triflate

In a dry box, ytterbium triflate (3.00 g) was added to a 100 mL round bottom flask equipped with a stirring bar. The flask was sealed with a rubber septum and removed from the dry box. After the attachment of a nitrogen bleed THF (20.00 mL) was added followed by pyruvonitrile (95%, 2.00 mL). After 60 minutes the polymerization was terminated by the addition of water (10 mL), THF (25 mL) and diethyl ether (25 mL). The resulting organic phase was separated, concentrated at reduced pressure and then dried under vacuum. Polymer yield: 1.44 g. GPC analysis: Mn = 52700, Mw = 67000, PD = 1.27 (PS STD.).

### EXAMPLE 97

### Polymerization of THF with Acetic Acid/ Acetic Anhydride and Yttrium Triflate

In a dry box, yttrium triflate (1.00 g) was added to an oven dried 100 mL RB flask equipped with a stirring bar. The flask was sealed with a rubber septum and removed from the dry box. After the attachment of a nitrogen bleed THF (20.00 mL), acetic acid (2.00 mL) and acetic anhydride (2.00 mL) were added via syringe. After 60 minutes the polymerization was terminated via the addition of water (25 mL), THF (50 mL) and ether (25 mL). The resulting organic phase was separated, concentrated at reduced pressure and then dried under vacuum. Polymer yield: 7.32 g. GPC analysis: Mn = 2470, Mw - 5250, PD = 2.13 (PS STD.).

### EXAMPLE 98

### Polymerization of THF with 11-Cyano-1-undecanoic Acid and Ytterbium Triflate

In a dry box, ytterbium triflate (10.00 g) and 11-cyano-1-undecanoic acid (5.00 g) were added to an oven dried 100 mL RB flask equipped with a stirring bar. The flask was sealed with a rubber septum and removed from the dry box. After the attachment of a nitrogen bleed THF (20.00 mL) was added via syringe. After 6 hrs. the polymerization was terminated via the addition of water (25 mL), THF (50 mL) and ether (25 mL). The resulting organic phase was separated, washed with 5% sodium bicarbonate (2 x 25 mL), concentrated at reduced pressure and then dried under vacuum. Polymer yield: 5.61 g.

### EXAMPLE 99

### Polymerization of THF with 4-Acetylbutyric Acid and Ytterbium Triflate

In a dry box, ytterbium triflate (10.00 g) was added to an oven dried 100 mL RB flask equipped with a stirring bar. The flask was sealed with a rubber septum and removed from the dry box. After the attachment of a nitrogen bleed THF (20.00 mL) and 4-acetylbutyric (1.00 mL) were added via syringe. After 90 minutes the polymerization was terminated via the addition of water (25 mL), THF (50 mL) and ether (25 mL). The resulting organic phase was separated, washed with 5% sodium bicarbonate (2 x 25 mL), concentrated at reduced pressure and then dried under vacuum. Polymer yield: 3.25 g.

### EXAMPLE 100

### Polymerization of THF with Glycolic Acid and Ytterbium Triflate

In a dry box, ytterbium triflate (10.00 g) and glycolic acid (99%, 1.00 g) were added to an oven dried 100 mL RB flask equipped with a stirring bar. The flask was sealed with a rubber septum and removed from the dry box. After the attachment of a nitrogen bleed THF (20.00 mL) was'added via syringe. After 90 minutes the polymerization was terminated via the addition of water (25 mL), THF (50 mL) and ether (25 mL). The resulting organic phase was separated, concentrated at reduced pressure and then dried under vacuum. Polymer yield: 4.76 g.

### EXAMPLE 101

### Preparation of Bismuth Triflate

BiCl₃ (630 mg, 2 mmol) was slurried in CH₂Cl₂ (20 mL). Triflic acid (900 mg, 6 mmol) was added dropwise, and the mixture was stirred overnight at room temperature. The solvent was removed to give 0.9 g of an off white solid. ¹⁹F NMR (DMSO-d₆): δ -77.3.

### EXAMPLE 102

### Polymerization of THF with Bismuth Triflate and Acetic Anhydride

In a dry box, bismuth triflate (0.5 g) was added to an oven dried 100 mL RB flask equipped with a stirring bar. The flask was sealed with a rubber septum and removed from the dry box. A nitrogen bleed was attached and THF (10.00 mL) and acetic anhydride (1.00 mL) were added. After stirring for 60 minutes the polymerization was terminated via the addition of water (25 mL), ether (25 mL) and THF (50 mL). The resulting organic phase was separated, concentrated at reduced pressure and then dried under vacuum. Polymer Yield: 5.71 g. GPC analysis: Mn = 8350, Mw = 12400, PD = 1.49 (PS STD.).

### EXAMPLE 103

### Preparation of Zr (OSO₂CF₃) ₄.Zr (OCOCH₃) ₄

Solid Zr(OTf)₄ (0.5 g) and Zr(CF₃CO₂)₄ (0.5 g) were mixed and THF (25 mL) was added. The mixture was stirred for 15 minutes at room temperature. The solvent was removed and 0.9 g of off white solid was collected. ¹⁹F NMR (CDCl₃) : δ -78.3, -76.2 (Zr(CF₃CO₂)₄ comes at δ -75.8).

### EXAMPLE 104

### Polymerization of THF with Zr (OSO₂CF₃) _{4.}Zr (OCOCH₃) ₄ and Acetic Anhydride

In a dry box, Zr(OSO₂CF₃)₄.Zr(OCOCH₃)₄ (0.5 g) was added to an oven dried 100 mL RB flask equipped with a stirring bar. The flask was sealed with a rubber septum and removed from the dry box. A nitrogen bleed was attached and THF (10.00 mL) and acetic anhydride (1.00 mL) were added. After stirring for 60 minutes the polymerization was terminated via the addition of water (25 mL), ether (25 mL) and THF (50 mL). The resulting organic phase was separated, concentrated at reduced pressure and then dried under vacuum. Polymer Yield: 5.01 g. GPC analysis: Mn = 6900, Mw = 10500, PD = 1.53 (PS STD.).

### EXAMPLE 105

### Preparation of Gold Triflate

AuBr₃ (0.90 g, 2.1 mmol) was slurried in CH₂Cl₂ (20 mL) and triflic acid (0.90 g, 6.3 mmol) was added dropwise. The mixture was stirred overnight at room temperature. The solvent was removed and 0.77 g of black solid was collected. ¹⁹F NMR (DMSO-d₆): δ -76.9.

### EXAMPLE 106

### Polymerization of THF with Gold(III) Triflate and Acetic Anhydride

In a dry box, gold(III) triflate (0.5 g) was added to an oven dried 100 mL RB flask equipped with a stirring bar. The flask was sealed with a rubber septum and removed from the dry box. A nitrogen bleed was attached and THF (10.00 mL) and acetic anhydride (1.00 mL) were added. After stirring for 60 minutes the polymerization was terminated via the addition of water (25 mL), ether (25 mL) and THF (50 mL). The resulting organic phase was separated, concentrated at reduced pressure and then dried under vacuum. Polymer Yield: 6.04 g. GPC analysis: Mn = 5240, Mw = 9060, PD = 1.73 (PS STD.).

### EXAMPLE 107

### Preparation of Y(OSO₂CF₃)₂Cl

Solid Y(OTf)₃ (540 mg, 1 mmol) and YCl₃ (98 mg, 0.5 mmol) were mixed, and this mixture was poured into stirred THF (30 mL). The mixture became warm as the solid dissolved. The solution was stirred for 15 min, and the THF was removed. ¹⁹F NMR (DMSO-d₆) : δ -77.3.

### EXAMPLE 108

### Polymerization of THF with Y(OSO₂CF₃)₂Cl and Acetic Anhydride

In a dry box, Y(OSO₂CF₃)₂Cl (0.5 g) was added to an oven dried 100 mL RB flask equipped with a stirring bar. The flask was sealed with a rubber septum and removed from the dry box. A nitrogen bleed was attached and THF (10.00 mL) and-acetic anhydride (1.00 mL) were added. After stirring for 60 minutes the polymerization was terminated via the addition of water (25 mL), ether (25 mL) and THF (50 mL). The resulting organic phase was separated, concentrated at reduced pressure and then dried under vacuum. Polymer Yield: 2.95 g. GPC analysis: Mn = 7390, Mw = 12800, PD = 1.73 (PS STD.).

### EXAMPLE 109

### Preparation of Y(OSO₂CF₃)Cl₂

Solid Y(OTf)₃ (540 mg, 1 mmol) and YCl₃ (390 mg, 2 mmol) were mixed, and this mixture was poured into stirred THF (30 mL). The mixture became warm as the solid dissolved. The solution was stirred for 15 min, and the THF was removed. ¹⁹F NMR (DMSO-d6): δ -77.2

### EXAMPLE 110

### Polymerization of THF with Y(OSO₂CF₃)Cl₂ and Acetic Anhydride

In a dry box, Y(OSO₂CF₃)Cl₂ (0.5 g) was added to an oven dried 100 mL RB flask equipped with a stirring bar. The flask was sealed with a rubber septum and removed from the dry box. A nitrogen bleed was attached and THF (10.00 mL) and acetic anhydride (1.00 mL) were added. After stirring for 60 minutes the polymerization was terminated via the addition of water (25 mL), ether (25 mL) and THF (50 mL). The resulting organic phase was separated, concentrated at reduced pressure and then dried under vacuum. Polymer Yield: 0.09 g.

### EXAMPLE 111

### Preparation of Ta(OSO₂CF₃)₄OCH₂CH₃

Ta(OEt)₅ (813 mg, 2 mmol) was dissolved in CH₂Cl₂ (20 mL). Triflic acid (1.5 g, 10 mol) was added dropwise and the solution stirred overnight at room temperature. The solvent was removed to produce a colorless oil. ¹H and ¹⁹F NMR show a mixture of compounds. ¹H NMR (CDCl₃): δ 1.85 (t), 1.9 (t), 4.1, (q), 4.15 (broad, q).

### EXAMPLE 112

### Polymerization of THF with Ta(OSO₂CF₃)₄OCH₂CH₃ and Acetic Anhydride

In a dry box, Ta(OSO₂CF₃)₄OCH₂CH₃ (0.5 g) was added to an oven dried 100 mL RB flask equipped with a stirring bar. The flask was sealed with a rubber septum and removed from the dry box. A nitrogen bleed was attached and THF (10.00 mL) and acetic anhydride (1.00 mL) were added. After stirring for 60 minutes the polymerization was terminated via the addition of water (25 mL), ether (25 mL), and THF (50 mL). The resulting organic phase was separated, concentrated at reduced pressure and then dried under vacuum. Polymer Yield: 6.29 g. GPC analysis: Mn = 2320, Mw = 5400, PD = 2.33 (PS STD.).

### EXAMPLE 113

### Preparation of Iron(III) Bis-triflate-acetylacetonate

Fe(acac)₃ (1.0 g, 2.8 mmol) was dissolved in CH₂Cl₂ (15 mL), and triflic acid (850 mg, 5.7 mmol) was added dropwise. The purple solution was stirred overnight at room temperature. The solvent was removed to give a dark oil.

### EXAMPLE 114

### Polymerization of THF with Iron(III) Bis-Triflate-Acetylacetonate and Acetic Anhydride

In a dry box, iron(III) bis-triflate-acetylacetonate (0.5 g) was added to an oven dried 100 mL RB flask equipped with a stirring bar. The flask was sealed with a rubber septum and removed from the dry box. A nitrogen bleed was attached and THF (10.00 mL) and acetic anhydride (1.00 mL) were added. After stirring for 60 minutes the polymerization was terminated via the addition of water (25 mL), ether (25 mL) and THF (50 mL). The resulting organic phase was separated, concentrated at reduced pressure and then dried under vacuum. Polymer Yield: 5.63 g. GPC analysis: Mn = 8330, Mw = 16100, PD = 1.94 (PS STD.).

### EXAMPLE 115

### Preparation of Ruthenium(III) Triflate

RuCl₃ (1.0 g, 4.6 mmol) was slurried in CH₂Cl₂ (20 mL) and triflic acid (2.0 g, 13.6 mmol) was added dropwise. The mixture was stirred at room temperature overnight. The solvent was removed and 1.15 g of black solid was collected. ¹⁹F NMR (CDCl₃): δ -76.7.

### EXAMPLE 116

### Polymerization of THF with Ruthenium(III) Triflate and Acetic Anhydride

In a dry box, ruthenium(III) triflate (0.5 g) was added to an oven dried 100 mL RB flask equipped with a stirring bar. The flask was sealed with a rubber septum and removed from the dry box. A nitrogen bleed was attached and THF (10.00 mL) and acetic anhydride (1.00 mL) were added. After stirring for 30 minutes the polymerization was terminated via the addition of water (25 mL), ether (25 mL) and THF (50 mL). The resulting organic phase was separated, concentrated at reduced pressure and then dried under vacuum. Polymer Yield: 5.25 g. GPC analysis: Mn = 7960, Mw = 12400, PD = 1.56 (PS STD.).

### EXAMPLE 117

### Preparation of Palladium(II) Triflate

PdCl₂ (1.0 g, 5.6 mmol) was slurried in CH₂Cl₂ (20 mL) and triflic acid (1.7 g, 11.3 mmol) was added dropwise. The mixture was stirred at room temperature overnight. The solvent was removed and 0.9 g of rust color solid was collected. ¹⁹F NMR (CDCl₃): δ -78.5.

### EXAMPLE 118

### Polymerization of THF with Palladium(II) Triflate and Acetic Anhydride

In a dry box, palladium(II) triflate (0.5 g) was added to an oven dried 100 mL RB flask equipped with a stirring bar. The flask was sealed with a rubber septum and removed from the dry box. A nitrogen bleed was attached and THF (10.00 mL) and acetic anhydride (1.00 mL) were added. After stirring for 60 minutes the polymerization was terminated via the addition of water (25 mL), ether (25 mL) and THF (50 mL). The resulting organic phase was separated, concentrated at reduced pressure and then dried under vacuum. Polymer Yield: 0.73 g. GPC analysis: Mn = 27100, Mw = 32500, PD = 1.20 (PS STD.).

### EXAMPLE 119

### Polymerization of THF with Niobium(V) Triflate and Acetic Anhydride

In a dry box, niobium(V) triflate (0.5 g) was added to an oven dried 100 mL RB flask equipped with a stirring bar. The flask was sealed with a rubber septum and removed from the dry box. A nitrogen bleed was attached and THF (10.00 mL) and acetic anhydride (1.00 mL) were added. After stirring for 60 minutes the polymerization was terminated via the addition of water (25 mL), ether (25 mL) and THF (50 mL). The resulting organic phase was separated, concentrated at reduced pressure and then dried under vacuum. Polymer Yield: 6.41 g. GPC analysis: Mn = 1580, Mw = 5810, PD = 3.67 (PS STD.).

### EXAMPLE 120

### Polymerization of THF with Tungsten(VI) Triflate and Acetic Anhydride

In a dry box, tungsten(VI) triflate (0.5 g) was added to an oven dried 100 mL RB flask equipped with a stirring bar. The flask was sealed with a rubber septum and removed from the dry box. A nitrogen bleed was attached and THF (10.00 mL) and acetic anhydride (1.00 mL) were added. After stirring for 60 minutes the polymerization was terminated via the addition of water (25 mL), ether (25 mL) and THF (50 mL). The resulting organic phase was separated, concentrated at reduced pressure and then dried under vacuum. Polymer Yield: 6.12 g. GPC analysis: Mn = 4430, Mw = 8330, PD = 1.88 (PS STD.).

### EXAMPLE 121

### Preparation of Rhenium(V) Triflate

ReCl₅ (1.0 g, 2.75 mmol) was slurried in CH₂Cl₂ (25 mL) and triflic acid (2.1 g, 13.7 mmol) was added dropwise. The mixture was stirred overnight at room temperature. The solvent was removed and 0.9 g of black solid was collected. ¹⁹F NMR (CDCl₃) : δ -74.4, -76.3 (small peak).

### EXAMPLE 122

### Polymerization of THF with Rhenium(V) Triflate and Acetic Anhydride

In a dry box, rhenium(V) triflate (0.5 g) was added to an oven dried 100 mL RB flask equipped with a stirring bar. The flask was sealed with a rubber septum and removed from the dry box. A nitrogen bleed was attached and THF (10.00 mL) and acetic anhydride (1.00 mL) were added. After stirring for 60 minutes the polymerization was terminated via the addition of water (25 mL), ether (25 mL) and THF (50 mL). The resulting organic phase was separated, concentrated at reduced pressure and then dried under vacuum. Polymer Yield: 5.60 g. GPC analysis: Mn - 7170, Mw = 13500, PD - 1.89 (PS STD.).

### EXAMPLE 123

### Preparation of Chromium(II) Triflate

CrCl₂ (0.62 g, 5 mmol) was slurried in CH₂Cl₂ (20 mL) and triflic acid (2.3 g, 15 mmol) was added dropwise. The mixture was stirred overnight at room temperature. The solvent was removed and 1.25 g of gray solid was collected. ¹⁹F NMR (DMSO-d₆): δ -76.65, -76.72.

### EXAMPLE 124

### Polymerization of THF with Chromium(II) Triflate and Acetic Anhydride

In a dry box, chromium(II) triflate (0.5 g) was added to an oven dried 100 mL RB flask equipped with a stirring bar. The flask was sealed with a rubber septum and removed from the dry box. A nitrogen bleed was attached and THF (10.00 mL) and acetic anhydride (1.00 mL) were added. After stirring for 60 minutes the polymerization was terminated via the addition of water (25 mL), ether (25 mL) and THF (50 mL). The resulting organic phase was separated, concentrated at reduced pressure and then dried under vacuum. Polymer Yield: 4.87 g. GPC analysis: Mn = 9210, Mw = 18800, PD = 2.05 (PS STD.).

### EXAMPLE 125

### Preparation of n-Cyclopentadienyl-tris(trifluoromethanesulfonato) zirconium

Cp*ZrCl₃ (1.0 g, 3 mmol) was slurried in CH₂Cl₂ (40 mL). THF (10.00 mL) was added to dissolve the yellow solid. Solid AgOTf (2.3 g, 9 mmol) was added; a white solid formed immediately. The mixture was stirred 15 min, and the orangish solution was filtered. The solvent was removed to produce an orangish oil. The material was crystallized from ether and 1.1 g of yellow solid was collected. ¹H NMR showed several peaks around 2 ppm. Coordinated THF is observed by ¹H NMR (near δ 3.5 and 1.2).

### EXAMPLE 126

### Polymerization of THF with n-pentamethylcyclopentadienyl-tris-(trifluoromethanesulfonato)zirconium and Acetic Anhydride

In a dry box, *n*-pentamethylcyclopentadienyl-tris(trifluoromethanesulfonato) zirconium (0.5 g) was added to an oven dried 100 mL RB flask equipped with a stirring bar. The flask was sealed with a rubber septum and removed from the dry box. A nitrogen bleed was attached and THF (10.00 mL) and acetic anhydride (1.00 mL) were added. After stirring for 60 minutes the polymerization was terminated via the addition of water (25 mL), ether (25 mL) and THF (50 mL). The resulting organic phase was separated, concentrated at reduced pressure and then dried under vacuum. Polymer Yield: 6.49 g. GPC analysis: Mn = 4350, Mw = 7930, PD = 1.82 (PS STD.).

### EXAMPLE 127

### Preparation of Strontium Triflate

SrCl₂ (790 mg, 5 mmol) was slurried in CH₂Cl₂ (20 mL) and triflic acid (1.5 g, 10 mmol) was added dropwise. The mixture was stirred overnight at room temperature. The solvent was removed and 1.7 g of white solid was collected. ¹⁹F NMR (DMSO-d₆): δ -77.4.

### EXAMPLE 128

### Polymerization of THF with Strontium Triflate and Acetic Anhydride

In a dry box, strontium triflate (0.5 g) was added to an oven dried 100 mL RB flask equipped with a stirring bar. The flask was sealed with a rubber septum and removed from the dry box. A nitrogen bleed was attached and THF (10.00 mL) and acetic anhydride (1.00 mL) were added. After stirring for 60 minutes the polymerization was terminated via the addition of water (25 mL), ether (25 mL) and THF (50 mL). The resulting organic phase was separated, concentrated at reduced pressure and then dried under vacuum. Polymer Yield: 5.44 G. GPC analysis: Mn - 6630, Mw = 11500, PD = 1.73 (PS STD.).

### EXAMPLE 129

### Preparation of Cp₂(OTf)Zr-O-Zr(OTf)Cp₂

Cp₂Zr(Cl)-O-(Cl)ZrCp₂ (1.3 g, 2.5 mmol) was dissolved in CH₂Cl₂ (40 mL), and AgOTf (1.3 g, 5 mmol) was added. The mixture was stirred over a weekend. The mixture was filtered and the solvent was removed. A white solid formed as the solvent evaporated. The mixture was filtered and the solid was washed with Et₂O. 1.4 g of white solid was collected. ¹H NMR (toluene-d₈) : δ 6.0 (s).

### EXAMPLE 130

### Polymerization of THF with Cp₂(OTf)Zr-O-Zr(OTf)Cp₂ and Acetic Anhydride

In a dry box, Cp₂(OTf)Zr-O-Zr(OTf)Cp₂ (0.5 g) was added to an oven dried 100 mL RB flask equipped with a stirring bar. The flask was sealed with a rubber septum and removed from the dry box. A nitrogen bleed was attached and THF (10.00 mL) and acetic anhydride (1.00 mL) were added. After stirring for 60 minutes the polymerization was terminated via the addition of water (25 mL), ether (25 mL) and THF (50 mL). The resulting organic phase was separated, concentrated at reduced pressure and then dried under vacuum. Polymer Yield: 1.84 g. GPC analysis: Mn = 22600, Mw = 28800, PD = 1.28 (PS STD.).

### EXAMPLE 131

### Polymerization of THF with Cp₂MeZr(THF)BPh₄ and Acetic Anhydride

In a dry box, Cp₂MeZr(THF)BPh₄ (0.5 g) was added to an oven dried 100 mL RB flask equipped with a stirring bar. The flask was sealed with a rubber septum and removed from the dry box. A nitrogen bleed was attached and THF (10.00 mL) and acetic anhydride (1.00 mL) were added. After stirring for 60 minutes the polymerization was terminated via the addition of water (25 mL), ether (25 mL) and THF (50 mL). The resulting organic phase was separated, concentrated at reduced pressure and then dried under vacuum. Polymer Yield: 0.78 g. GPC analysis: Mn - 4840, Mw = 7390, PD = 1.53 (PS STD.).

### EXAMPLE 132

### Preparation of Bis- (n-Cyclopentadienyl)bis(trifluoromethanesulfonato)molybdenum

Solid Cp₂MoCl₂ (500 mg, 1.7 mmol) and AgOTf (0.91 g, 3.5 mmol) were mixed and CH₂Cl₂ (30 mL). The mixture stirred overnight at room temperature. The white solid was filtered off and the solvent was evaporated to give 300 mg of a green solid. ¹H NMR (CDCl₃) : δ 6.4 (s).

### EXAMPLE 133

### Polymerization of THF with Bis-(n-Cyclopentadienyl)bis-(trifluoromethanesulfonato)molybdenum and Acetic Anhydride

In a dry box, bis-(*n*-Cyclopentadienyl)-bis-(trifluoromethanesulfonato)molybdenum (0.275 g) was added to an oven dried 100 mL RB flask equipped with a stirring bar. The flask was sealed with a rubber septum and removed from the dry box. A nitrogen bleed was attached and THF (10.00 mL) and acetic anhydride (1.00 mL) were added. After stirring for 60 minutes the polymerization was terminated via the addition of water (25 mL), ether (25 mL) and THF (50 mL). The resulting organic phase was separated, concentrated at reduced pressure and then dried under vacuum. Polymer Yield: 0.77 g. GPC analysis: Mn = 15000, Mw = 21600, PD = 1.44 (PS STD.).

### EXAMPLE 134

### Preparation of Bis (trifluoromethanesulfonato)-bis (acetylacetonate) zirconium

Zr(acac)₄ (1.46 g, 3 mmol) was dissolved in CH₂Cl₂ (5 mL). A solution of triflic acid (0.9 g, 6 mmol) in CH₂Cl₂ (1 mL) was added to the Zr(acac)₄ solution. The solution was stirred 2 hours at room temperature. The solvent was removed and 1.79 g of yellow solid was collected. ¹⁹F NMR (CDCl₃) : δ -78.4; ¹H NMR (CDCl₃) : δ 2.15 (s), 5.82 (broad).

### EXAMPLE 135

### Polymerization of THF with Bis (trifluoromethanesulfonato)-bis (acetylacetonate) zirconium and Acetic Anhydride

In a dry box, bis (trifluoromethanesulfonato)bis (acetylacetonate) zirconium (0.50 g) was added to an oven dried 100 mL RB flask equipped with a stirring bar. The flask was sealed with a rubber septum and removed from the dry box. A nitrogen bleed was attached and THF (10.00 mL) and acetic anhydride (1.00 mL) were added. After stirring for 60 minutes the polymerization was terminated via the addition of water (25 mL), ether (25 mL) and THF (50 mL). The resulting organic phase was separated, concentrated at reduced pressure and then dried under vacuum. Polymer Yield: 5.27 g. GPC analysis: Mn = 13400, Mw = 20200, PD = 1.51 (PS STD.).

### EXAMPLE 136

### Preparation of Yttrium Bis (trifluoromethanesulfonato)-2,2,6,6-tetramethyl-3,5-heptanedionate

(t-Buacac)₃Y (0.64 g, 1 mmol) was dissolved in CH₂Cl₂ (5 mL). A solution of triflic acid (0.3 g, 2 mmol) in CH₂Cl₂ (1 mL) was added and the solution was stirred 2 hours at room temperature. The solvent was removed and a white solid was collected. ¹H NMR (CDCl₃). δ 1.2; 1.1 (broad s); ¹⁹F NMR (CDCl₃): δ -78.4.

### EXAMPLE 137

### Polymerization of THF with Yttrium Bis (trifluoromethanesulfonato)-2,2,6,6-tetramethyl-3,5-heptanedionate and Acetic Anhydride

In a dry box, yttrium bis (trifluoromethanesulfonato)-2,2,6,6-tetramethyl-3,5-heptanedionate (0.50 g) was added to an oven dried 100 mL RB flask equipped with a stirring bar. The flask was sealed with a rubber septum and removed from the dry box. A nitrogen bleed was attached and THF (10.00 mL) and acetic anhydride (1.00 mL) were added. After stirring for 60 minutes the polymerization was terminated via the addition of water (25 mL), ether (25 mL) and THF (50 mL). The resulting organic phase was separated, concentrated at reduced pressure and then dried under vacuum. Polymer Yield: 1.26 g. GPC analysis: Mn = 17200, Mw = 25300, PD = 1.47 (PS STD.).

### EXAMPLE 138

### Preparation of Yttrium Trifluoromethanesulfonato-bis (2,2,6,6-tetramethyl-3,5-heptanedionate)

(t-Buacac)₃Y (0.64 g, 1 mmol) was dissolved in CH₂Cl₂ (5 mL). A solution of triflic acid (0.15 g, 1 mmol) in CH₂Cl₂ (1 mL) was added dropwise and the solution was stirred 2 h at room temperature. The solvent was removed and a white solid was collected. ¹H NMR (CDCl₃) : δ 1.15, 1.02; ¹⁹F NMR (CDCl₃) : δ -78.6 (small, broad), -76.7.

### EXAMPLE 139

### Polymerization of THF with Yttrium Trifluoromethanesulfonato-bis (2,2,6,6-tetramethyl-3,5-heptanedionate) and Acetic Anhydride

In a dry box, yttrium trifluoromethanesulfonatobis (2,2,6,6-tetramethyl-3,5-heptanedionate) (0.50 g) was added to an oven dried 100 mL RB flask equipped with a stirring bar. The flask was sealed with a rubber septum and removed from the dry box. A nitrogen bleed was attached and THF (10.00 mL) and acetic anhydride (1.00 mL) were added. After stirring for 60 minutes the polymerization was terminated via the addition of water (25 mL), ether (25 mL) and THF (50 mL). The resulting organic phase was separated, concentrated at reduced pressure and then dried under vacuum. Polymer Yield: 0.16 g.

### EXAMPLE 140

### Preparation of VO(OTf)ₙ(OCHMe₂)₃₋ₙ

V(O) (OnPr)₃ (1.2 g, 5 mmol) was dissolved in CH₂Cl₂ (30 mL). Triflic acid (2.2 g, 15 mmol) was added dropwise to produce a dark red solution. The solvent was removed and a dark oil was produced.

### EXAMPLE 141

### Polymerization of THF with VO(OTf)ₙ(OCHMe₂)₃₋ₙ and Acetic Anhydride

In a dry box, VO(OTf)ₙ(OCHMe₂)₃₋ₙ (0.50 g) was added to an oven dried 100 mL RB flask equipped with a stirring bar. The flask was sealed with a rubber septum and removed from the dry box. A nitrogen bleed was attached and THF (10.00 mL) and acetic anhydride (1.00 mL) were added. After stirring for 60 minutes the polymerization was terminated via the addition of water (25 mL), ether (25 mL) and THF (50 mL). The resulting organic phase was separated, concentrated at reduced pressure and then dried under vacuum. Polymer Yield: 6.07 g. GPC analysis: Mn = 4770, Mw = 29110, PD = 1.91 (PS STD.).

### EXAMPLE 142

### Preparation of Silicon Triflate

SiCl₄ (3 g, 17.6 mmol) was dissolved in CH₂Cl₂ (75 mL) and triflic acid (10.6 g, 70.7 mmol) was added dropwise. The mixture was stirred at room temperature over the weekend. The solvent was removed and 4.8 g of brown liquid was collected. ¹⁹F NMR (DMSO-d₆): δ -76.4 (intense), small broad peaks at -77.8 and -77.95.

### EXAMPLE 143

### Polymerization of THF with Silicon Triflate and Acetic Anhydride

In a dry box, silicon triflate (0.50 g) was added to an oven dried 100 mL RB flask equipped with a stirring bar. The flask was sealed with a rubber septum and removed from the dry box. A nitrogen bleed was attached and THF (10.00 mL) and acetic anhydride (1.00 mL) were added. After stirring for 60 minutes the polymerization was terminated via the addition of water (25 mL), ether (25 mL) and THF (50 mL). The resulting organic phase was separated, concentrated at reduced pressure and then dried under vacuum. Polymer Yield: 7.76 g. GPC analysis: Mn = 1450, Mw = 3170, PD = 2.18 (PS STD.).

### EXAMPLE 144

### Polymerization of 1,3-Dioxolane with Ytterbium Triflate

In a dry box, ytterbium triflate (1.5 g) was added to an oven dried 100 mL RB flask equipped with a stirring bar. The flask was sealed with a rubber septum and removed from the dry box. After the attachment of nitrogen bleeds 1,3-dioxolane (10.00 mL) was added to the flask. After 60 minutes the polymerization was terminated via the addition of water (25 mL), THF (50 mL) and ether (25 mL). The resulting aqueous phase was separated, concentrated at reduced pressure and then dried under vacuum. Yield: 8.89 g (no attempt was made to remove the catalyst from the polymer). GPC analysis (PS STD.). Mn = 4170, Mw = 8550, PD = 2.05.

### EXAMPLE 145

### Polymerization of 1,3,5-Trioxane with Ytterbium Triflate

In a dry box, ytterbium triflate (1.5 g) was added to an oven dried 100 mL RB flask equipped with a stirring bar. In a separate 100 mL RB flask 1,3,5-trioxane (20.00 g) was added. The flasks were sealed with a rubber septum and removed from the dry box. To the flask containing the trioxane cyclohexane (20 mL) was added, and the resulting mixture heating to 60°C via an oil bath until an homogeneous solution resulted. This solution (20.00 mL) was then added via syringe to the flask with ytterbium triflate at this temperature. The resulting mixture was then placed in an oil bath maintained at 60°C . After 60 minutes, the polymerization was terminated by the addition of water (25 mL) and ether (25 mL). The resulting solid was separated and dried under vacuum, giving 4.74 g of polymer.

### EXAMPLE 146

### Polymerization of THF with N-Acetylglycine and Ytterbium Triflate

In a dry box, ytterbium triflate (10.00 g) and N-acetylglycine (2.00 g) were added to an oven dried 100 mL RB flask equipped with a stirring bar. The flask was sealed with a rubber septum and removed from the dry box. After the attachment of a nitrogen bleed THF (20.00 mL) was added via syringe. After 180 minutes the polymerization was terminated via the addition of water (25 mL), THF (50 mL) and ether (25 mL). The resulting organic phase was separated, concentrated at reduced pressure and then dried under vacuum. Polymer yield: 5.27 g (29.7%). GPC analysis: Mn = 20500, Mw = 43700, PD = 2.13 (PS STD.).

### EXAMPLE 147

### Polymerization of THF with N-Acetyl-DL-tryptophan and Ytterbium Triflate

In a dry box, ytterbium triflate (5.00 g) and N-acetyl-DL-tryptophan (1.00 g) were added to an oven dried 100 mL RB flask equipped with a stirring bar. The flask was sealed with a rubber septum and removed from the dry box. After the attachment of a nitrogen bleed THF (20.00 mL) was added via syringe.. After 26 hrs. the polymerization was terminated via the addition of water (25 mL), THF (50 mL) and ether (25 mL). The resulting organic phase was separated, concentrated at reduced pressure and then dried under vacuum. Polymer yield: 7.13 g. GPC analysis: Mn = 40200, Mw = 92100, PD = 2.29 (PS STD.).

## Claims

1. A process for the polymerization of cyclic ethers, comprising, contacting one or more oxiranes, oxetanes, tetrahydrofurans, oxepanes, 1,3-dioxolanes or 1,3,5-trioxanes with a compound of the formula MZₛ·Qₜ, and an accelerator selected from the group consisting of carboxylic acids whose pKa in water is less than 6, carboxylic anhydrides and acyl halides, wherein:
M is a metal selected from the group consisting of cobalt, vanadium, niobium, tungsten, strontium, barium, scandium, yttrium, thulium and the other rare earth metals, titanium, zirconium, hafnium, chromium, silicon, molybdenum, tantalum, rhenium, iron, ruthenium, osmium, rhodium, iridium, palladium, platinum, gold, zinc, copper, mischmetall, cadmium, mercury, indium, germanium, tin, lead, arsenic, antimony and bismuth;
at least one of Z is an anion of the formula ⁻OSO₂R⁵, wherein R⁵ is perfluoroalkyl containing 1 to 12 carbon atoms or part of a fluorinated polymer wherein the carbon atoms alpha and beta to the sulfonate group are together bonded to at least four fluorine atoms, or tetraphenylborate, and the remainder of Z is oxo or one or more monovalent anions;
s is 2 when M is copper, strontium, barium, cobalt, rhodium,, iridium, palladium, platinum, chromium, zinc, cadmium or mercury;
s is 3 when M is scandium, yttrium, thulium or other rare earth metal, arsenic, antimony, bismuth, gold, iron, ruthenium, osmium, indium or mischmetall;
s is 4 when M is titanium, zirconium, hafnium, silicon, molybdenum, germanium, tin, or lead;
s is 5 when M is rhenium, vanadium, niobium or tantalum;
s is 6 when M is tungsten;
Q is a neutral ligand;
t is 0 or an integer of 1 to 6;
and provided that each oxo group present as part of Z is considered to account for two of s and wherein the process is carried out at -80°C to 130°C, and said compound of the formula MZₛ·Qₜ remains as a perfluoroalkylsulfonate salt during said polymerization.

2. The process as recited in Claim 1 wherein said cyclic ether is one or more of said tetrahydrofurans, oxepanes, 1,3-dioxolanes or 1,3,5-trioxanes.

3. The process as recited in Claim 2 wherein M is a metal selected from the group consisting of strontium, barium, scandium, yttrium, the rare earth metals, titanium, zirconium, hafnium, chromium, molybdenum, tantalum, rhenium, iron, ruthenium, osmium, rhodium, iridium, palladium, platinum, gold, zinc, cadmium, mercury, germanium, tin, lead, arsenic, antimony, bismuth, copper and mischmetall.

4. The process as recited in Claim 3 wherein said cyclic ether comprises the formula 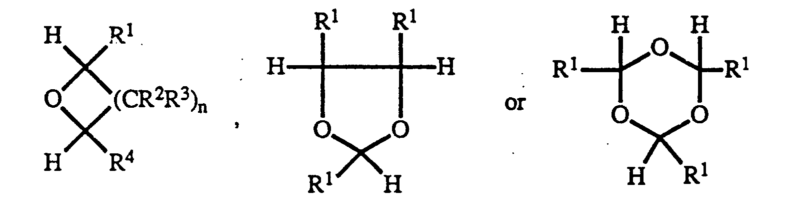 wherein:
each R¹, R², R³ and R⁴ is independently hydrogen or hydrocarbyl containing 1 to 20 carbon atoms; and
n is 2 or 4.

5. The process as recited in Claim 4 wherein n is 2 and R¹, R⁴ and all of R² and R³ are hydrogen.

6. The process as recited in Claim 4 wherein n is 2 and R¹ and R⁴ are each hydrogen, one of R² is hydrogen, the other R² is methyl, and both R³ are hydrogen.

7. The process as recited in Claim 3 wherein R⁵ is trifluoromethyl or perfluoroalkyl.

8. The process as recited in Claim 5 wherein M is strontium, scandium, yttrium, the rare earth metals, titanium, zirconium, hafnium, vanadium, niobium, tantalum, chromium, molybdenum, tungsten, rhenium, iron, ruthenium, palladium, copper, gold, zinc, tin, bismuth or mischmetall.

9. The process as recited in Claim 1 wherein said carboxylic acid is trifluoroacetic acid, formic, acetic, cyanoacetic, nitropropionic, acrylic, methacrylic acid, N-acetylglycine or N-acetyltryptophan.

10. The process as recited in Claim 1 wherein said carboxylic anhydride is acetic anhydride or trifluoroacetic anhydride.

## Patentansprüche

1. Verfahren für die Polymerisation cyclischer Ether, welches Verfahren das Kontaktieren von einem oder mehreren Oxiranen, Oxetanen, Tetrahydrofuranen, Oxepanen, 1,3-Dioxolanen oder 1,3,5-Trioxanen mit einer Verbindung der Formel MZ_{S}·Qₜ und einem Beschleuniger umfasst, ausgewählt aus der Gruppe, bestehend aus Carbonsäuren, deren pKa in Wasser kleiner ist als 6, Carbonsäureanhydriden und Acylhalogeniden, worin sind:
M ein Metall, ausgewählt aus der Gruppe, bestehend aus Kobalt, Vanadium, Niob, Wolfram, Strontium, Barium, Scandium, Yttrium, Thulium und die anderen Seltenerdmetallen, Titan, Zirconium, Hafnium, Chrom, Silicium, Molbdän, Tantal, Rhenium, Eisen, Ruthenium, Osmium, Rhodium, Iridium, Palladium, Platin, Gold, Zink, Kupfer, Mischmetall, Cadmium, Quecksilber, Indium, Germanium, Zinn, Blei, Arsen, Antimon und Bismut;
mindestens eines von Z ist ein Anion der Formel ⁻OS₂R⁵, worin R⁵ Perfluoralkyl ist und 1 bis 12 Kohlenstoffatome enthält, oder ein Teil eines fluorierten Polymers ist, worin die Kohlenstoffatome alpha und beta zu der Sulfonat-Gruppe gemeinsam an mindestens vier Fluoratomen gebunden sind, oder Tetraphenylborat; und der Rest von Z ist Oxo oder ein oder mehrere einwertige Ionen;
s 2, wenn M Kupfer, Strontium, Barium, Kobalt, Rhodium, Iridium, Palladium, Platin, Chrom, Zink, Cadmium oder Quecksilber ist;
s 3, wenn M Scandium, Yttrium, Thulium oder anderes Seltenerdmetall, Arsen, Antimon, Bismut, Gold, Eisen, Ruthenium, Osmium, Indium oder Mischmetall ist;
s 4, wenn M Titan, Zirconium, Hafnium, Silicium, Molybdän, Germanium, Zinn oder Blei ist;
s 5, wenn M Rhenium, Vanadium, Niob oder Tantal ist;
s 6, wenn M Wolfram ist;
Q ein neutraler Ligand;
t Null oder eine ganze Zahl von 1 bis 6;
und vorausgesetzt, dass jede als Teil von Z vorhandene Oxo-Gruppe für zwei s zählt; und wobei das Verfahren bei -80°C bis 130°C ausgeführt wird und die Verbindung der Formel MZ_{S}·Qₜ während dieser Polymerisation ein Perfluoralkylsulfonat-Salz bleibt.

2. Verfahren nach Anspruch 1, bei welchem der cyclische Ether einer oder mehrere der Tetrahydrofurane, Oxepane, 1,3-Dioxolane oder 1,3,5-Trioxane ist.

3. Verfahren nach Anspruch 2, bei welchem M ein Metall ist, ausgewählt aus der Gruppe, bestehend aus Strontium, Barium, Scandium, Yttrium, Seltenerdmetallen, Titan, Zirconium, Hafnium, Chrom, Molybdän, Tantal, Rhenium, Eisen, Ruthenium, Osmium, Rhodium, Iridium, Palladium, Platin, Gold, Zink, Cadmium, Quecksilber, Germanium, Zinn, Blei, Arsen, Antimon, Bismut, Kupfer und Mischmetall.

4. Verfahren nach Anspruch 3, bei welchem der cyclische Ether die Formel hat: 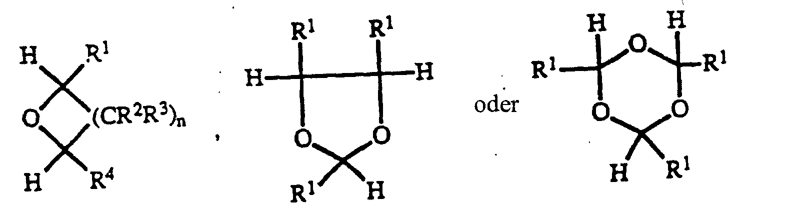 worin sind:
jedes R¹, R², R³ und R⁴ unabhängig Wasserstoff oder Hydrocarbyl, das 1 bis 20 Kohlenstoffatome enthält; und
n 2 oder 4.

5. Verfahren nach Anspruch 4, bei welchem n 2 ist und R¹, R⁴ und alle R² und R³ Wasserstoff sind.

6. Verfahren nach Anspruch 4, bei welchem n 2 ist und R¹ und R⁴ jedes Wasserstoff sind, eines von R² Wasserstoff ist, das andere R² Methyl ist und beide R³ Wasserstoff sind.

7. Verfahren nach Anspruch 3, bei welchem R⁵ Trifluormethyl oder Perfluoralkyl ist.

8. Verfahren nach Anspruch 5, bei welchem M Strontium, Scandium, Yttrium, Seltenerdmetall, Titan, Zirconium, Hafnium, Vanadium, Niob, Tantal, Chrom, Molybdän, Wolfram, Rhenium, Eisen, Ruthenium, Palladium, Kupfer, Gold, Zink, Zinn, Bismut oder Mischmetall ist.

9. Verfahren nach Anspruch, bei welchem die Carbonsäure Trifluoressigsäure, Ameisensäure, Essigsäure, Cyanessigsäure, Nitropropansäure, Acrylsäure, Methacrylsäure, N-Acetylglycin oder N-Acetyltryptophan ist.

10. Verfahren nach Anspruch 1, bei welchem das Carbonsäureanhydrid Essigsäureanhydrid oder Trifluoressigsäureanhydrid ist.

## Revendications

1. Procédé pour la polymérisation d'éthers cycliques, comprenant, la mise en contact d'une ou plusieurs oxirannes, oxétannes, tétrahydrofurannes, oxépannes, 1,3-dioxolannes ou 1,3,5-trioxannes avec un composé de formule MZₛ.Qₜ, et un accélérateur sélectionné parmi le groupe se composant d'acides carboxyliques dont le pKa dans l'eau est inférieur à 6, d'anhydrides carboxyliques et d'halogénures d'acyle :
M étant un métal sélectionné parmi le groupe se composant de cobalt, vanadium, niobium, tungstène, strontium, baryum, scandium, yttrium, thulium et les autres métaux de terres rares, titane, zirconium, hafnium, chrome, silicium, molybdène, tantale, rhénium, fer, ruthénium, osmium, rhodium, iridium, palladium, platine, or, zinc, cuivre, mischmétal, cadmium, mercure, indium, germanium, étain, plomb, arsenic, antimoine et bismuth ;
Au moins l'un des Z étant un anion de formule -OSO₂R⁵ dans laquelle R⁵ est un perfluoroalkyle contenant 1 à 12 atomes de carbone ou une partie d'un polymère fluoré dans lesquels les atomes de carbone alpha et bêta à côté du groupement sulfonate sont ensemble liés à au moins 4 atomes de fluor, ou un tétraphénylborate, et le reste des Z étant oxo ou un ou plusieurs anions monovalents ;
s étant 2 lorsque M est cuivre, strontium, baryum, cobalt, rhodium, iridium, palladium, platine, chrome, zinc, cadmium ou mercure ;
s étant 3 lorsque M est scandium, yttrium, thulium ou un autre métal de terres rares, arsenic, antimoine, bismuth, or, fer, ruthénium, osmium, indium ou mischmétal ;
s étant 4 lorsque M est titane, zirconium, hafnium, silicium, molybdène, germanium, étain ou plomb ;
s étant 5 lorsque M est rhénium, vanadium, niobium ou tantale ;
s étant 6 lorsque M est tungstène ;
Q étant un ligand neutre ;
t étant O ou un nombre entier de 1 à 6 ;
et sous réserve que chaque groupement oxo présent en tant que partie des Z soit considéré de façon à compter pour 2 de s et le procédé étant exécuté à -80°C à 130°C, et ledit composé de formule MZₛ.Qₜ demeurant un sel de perfluoroalkylsulfonate pendant ladite polymérisation.

2. Procédé selon la revendication 1 dans lequel ledit éther cyclique est un ou plusieurs desdits tétrahydrofurannes, oxépannes, 1,3-dioxolannes ou 1,3,5-trioxannes.

3. Procédé selon la revendication 2 dans lequel M est un métal sélectionné parmi le groupe se composant de strontium, baryum, scandium, yttrium, les métaux de terres rares, titane, zirconium, hafnium, chrome, molybdène, tantale, rhénium, fer, ruthénium, osmium, rhodium, iridium, palladium, platine, or, zinc, cadmium, mercure, germanium, étain, plomb, arsenic, antimoine, bismuth, cuivre et mischmétal.

4. Procédé selon la revendication 3, dans lequel ledit éther cyclique est de formules 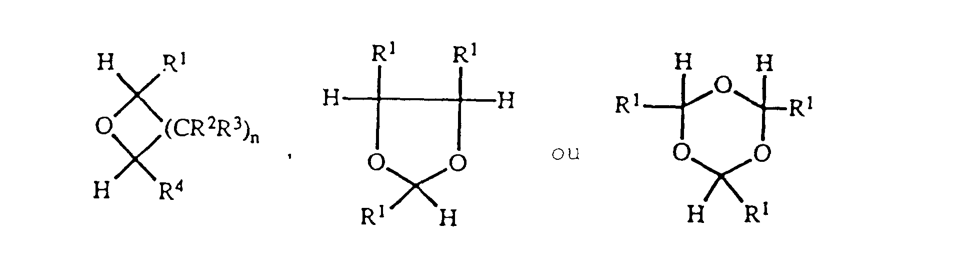 dans lesquelles :
chaque R¹, R², R³, et R⁴ est de façon indépendante un hydrogène ou un hydrocarbyle contenant 1 à 20 atomes de carbone ; et
n est 2 ou 4.

5. Procédé selon la revendication 4, dans lequel n est 2 et R¹, R⁴ et tous les R² et R³ sont un hydrogène.

6. Procédé selon la revendication 4, dans lequel n est 2 et R¹ et R⁴ sont chacun un hydrogène, un parmi les R² est un hydrogène, l'autre R² est un méthyle, et les deux R³ sont hydrogène.

7. Procédé selon la revendication 3 dans lequel R⁵ est un trifluorométhyle ou un perfluoroalkyle.

8. Procédé selon la revendication 5 dans lequel M est strontium, scandium, yttrium, les métaux de terres rares, titane, zirconium, hafnium, vanadium, niobium, tantale, chrome, molybdène, tungstène, rhénium, fer, ruthénium, palladium, cuivre, or, zinc, étain, bismuth ou mischmétal.

9. Procédé selon la revendication 1 dans lequel ledit acide carboxylique est l'acide trifluoroacétique, l'acide formique, l'acide acétique, l'acide cyanoacétique, l'acide nitropropionique, l'acide acrylique, l'acide méthacrylique, la N-acétylglycine ou le N-acétyltryptophanne.

10. Procédé selon la revendication 1 dans lequel ledit anhydride carboxylique est l'anhydride acétique ou l'anhydride trifluoroacétique.
